# EUROPEAN PATENT SPECIFICATION

(11) **EP 1 747 287 B1**
(45) Date of publication and mention of the grant of the patent: **23.10.2013**
(21) Application number: 05744681.7
(22) Date of filing: 18.05.2005
(51) Int. Cl.: C12Q 1/68

(54) **GENOTOXIC TESTING**
GENOTOXISCHES TESTEN
EPREUVES DE GENOTOXICITE

(30) Priority: 20.05.2004 GB 0411198
(43) Date of publication of application: 31.01.2007
(73) Proprietor: Gentronix Limited, Manchester M13 9NT (GB)
(72) Inventor: HASTWELL, Paul, Bedfordshire LU7 3DS (GB); WALMSLEY, Richard, Faculty of Lice Sciences, PO Box 88, Sackville Street, Manchester (GB)
(74) Representative: Banford, Paul Clifford
(86) International application number: PCT/GB2005/001913
(87) International publication number: WO 2005/113802

(56) References cited:
- WO-A-98/44149
- US-A1- 2001 007 768
- US-A1- 2003 049 690
- US-A1- 2003 134 288
- AKUTSU N ET AL: "Regulation of gene expression by 1alpha,25-dihydroxyvitamin D3 and its analog EB1089 under growth-inhibitory conditions in squamous carcinoma cells" MOLECULAR ENDOCRINOLOGY, BALTIMORE, MD, US, vol. 15, no. 7, July 2001 (2001-07), pages 1127-1139, XP002227920 ISSN: 0888-8809
- ZHENG X ET AL: "Inhibition of NF-kappaB stabilizes gadd45alpha mRNA" BIOCHEMICAL AND BIOPHYSICAL RESEARCH COMMUNICATIONS, ACADEMIC PRESS INC. ORLANDO, FL, US, vol. 329, no. 1, 1 April 2005 (2005-04-01), pages 95-99, XP004757002 ISSN: 0006-291X

### SEQUENCE LISTING

<110> Gentronix Gentronix Ltd
<120> GENOTOXIC TESTING
<130> PCB/AH/P89625PWO
<160> 5
<170> PatentIn version 3.1
<210> 1
   <211> 3516
   <212> DNA
   <213> Homo sapiens
<220>
   <221> GADD45 alpha promoter
   <222> (4)..(2254)
   <223>
<220>
   <221> GADD45 alpha 5' UTR
   <222> (2255)..(2549)
   <223>
<220>
   <221> EGFP gene
   <222> (2550)..(3278)
   <223>
<220>
   <221> SV40 polyA
   <222> (3432)..(3482)
   <223>
<400> 1
   atcagatctt gggtggggca ctttaggact gtggttcatt tgaattggtg taaacaatac     60
   accggttcta ctgtcctaca gcctccattc agatgactga agtcatggga ctttcagcat    120
   agctagctga tgacagtgca tactattttg tcccaaaatc cagttcaagc atggacatac    180
   caataagagc ctaagctctt taaaggcaaa ggaccaggaa ttgtacagtt cttggtatag    240
   aagaagacag gcaaaagtgt ttttgaacta acgttaaatg tgcaatatgt tagaattcat    300
   gcaatgcaca ggactgcagg attctgatat cttatttaac tctcaaattc tattcaactc    360
   aataaacctt gactgtgctt ctactaaatg caggtattgt actaggagct gaggacacca    420
   aactgatgaa gtccttgctg tcaagaaact cacatgattc cctaattctt tgtcagcttg    480
   ctgtgatcac attttcttcc caagaacctc taagaaatgc ctagtggata gaaccttgga    540
   gttccacgga acatattaac aatcgccaaa tgatgactca ggctagattg tgtaattcag    600
   gttttgtctg caaaactgaa aatgcttcgg taacctacct aaatttcaat gttgaggaat    660
   tctttaagaa agacatcaaa tgttaagatt taaggcatag atatgagata catagtcatg    720
   cttaggtgaa ttatgcactg accatgacca tttctttact caaatgttgt ccatggctga    780
   caacacagtg aaaaaatgag tgcaaaatga caactcaaat aaatgaacca gaaaacctat    840
   cacttttctt ttccaccaaa ttaagatcaa gagagctgga gaatattttg tctagagtga    900
   taaaaacata agggtgcaaa acttccaggt acctttgcag aaattacttc tgtgaccttt    960
   ggctgtacag caaccttaat aatgcaagca ctgttttgaa tgcaagcatg tgggagccat   1020
   tttcaccact tttgatgact tcagtaggtt taagaaatgt ttttgctttt attgcataaa   1080
   ccataaaaca aaggaaggga cttttgaact actcagtgag agtctatata ttaaagtttg   1140
   tttttcaaaa atgtgtaact accatttgca gttttaaagg tctgctttcc acctacaagt 1200
   tgccattatc tcaaaggtga aattttagca tatgactaaa aacttcctat agttacagct 1260
   tcatgattca gcatctaaca tcaataattc acagtgagat cataggaggc tctctgtgga 1320
   aggtaacgac atacatacgt taggaaagga agcttagggc atatcgagag cattttgaat 1380
   ttagacttgt gggctgtgtg ggtgtcagat ggttgtctct cagctggtgg gcgtccagaa 1440
   ggatccttgt ttgggcaagg ctctttgaga aaggagaatc tgggttgcca gggattccca 1500
   catgtggtca ccagctcccc acgcagacca gctcacgatt tcccagttac accgggcagg 1560
   tgggaaaccg ttctgctttc tgtggaaaag attctaactt ggttccctgc catccctgaa 1620
   tacaaacggg ttggtttttc ttttttcagc ttccaaccct tgcagctttc caaaaataaa 1680
   tcaaaccagc catcagggca ccgaaataat actactgcta ataagcagct tcgcctagac 1740
   ttagataaac aacacttctg aggtaaactt tgccccggag gtctggagac acttttttaa 1800
   tgtaacctgc ttactaataa ttactagact tcagtgcatt aaccctggaa atagatttta 1860
   atagccaccc cttaaaacaa aagacatgaa aagataataa gaaaaaagtg ccgcaactat 1920
   tatagaaaaa cacttggcag cctgcttcag cccaagctga ggccacctct agcctctgct 1980
   aaagcccccc actcccaatg gtccccgcca accggataag agtgcgcgcg ggacccgcct 2040
   tcccctctcg gcaccgcccc cgcccccgcc ccctcggctc gcctcccgcg tggctcctcc 2100
   cttttccgct cctctcaacc tgactccagg agctggggtc aaattgctgg agcaggctga 2160
   tttgcatagc ccaatggcca agctgcatgc aaatgaggcg gaaggtggtt ggctgagggt 2220
   tggcaggata accccggaga gcggggccct ttgtcctcca gtggctggta ggcagtggct 2280
   gggaggcagc ggcccaatta gtgtcgtgcg gcccgtggcg aggcgaggtc cggggagcga 2340
   gcgagcaagc aaggcgggag gggtggccgg agctgcggcg gctggcacag gaggaggagc 2400
   ccgggcgggc gaggggcggc cggagagcgc cagggcctga gctgccggag cggcgcctgt 2460
   gagtgagtgc agaaagcagg cgcccgcgcg ctagccgtgg caggagcagc ccgcacgccg 2520
   cgctctctcc ctgggcgacc tgcagtttgc aatatggcgc gcctggtgag caagggcgag 2580
   gagctgttca ccggggtggt gcccatcctg gtcgagctgg acggcgacgt aaacggccac 2640
   aagttcagcg tgtccggcga gggcgagggc gatgccacct acggcaagct gaccctgaag 2700
   ttcatctgca ccaccggcaa gctgcccgtg ccctggccca ccctcgtgac caccctgacc 2760
   tacggcgtgc agtgcttcag ccgctacccc gaccacatga agcagcacga cttcttcaag 2820
   tccgccatgc ccgaaggcta cgtccaggag cgcaccatct tcttcaagga cgacggcaac 2880
   tacaagaccc gcgccgaggt gaagttcgag ggcgacaccc tggtgaaccg catcgagctg 2940
   aagggcatcg acttcaagga ggacggcaac atcctggggc acaagctgga gtacaactac   3000
   aacagccaca acgtctatat catggccgac aagcagaaga acggcatcaa ggtgaacttc   3060
   aagatccgcc acaacatcga ggacggcagc gtgcagctcg ccgaccacta ccagcagaac   3120
   acccccatcg gcgacggccc cgtgctgctg cccgacaacc actacctgag cacccagtcc   3180
   gccctgagca aagaccccaa cgagaagcgc gatcacatgg tcctgctgga gttcgtgacc   3240
   gccgccggga tcactctcgg catggacgag ctgtacaagt aaagcggccg cgactctaga   3300
   tcataatcag ccataccaca tttgtagagg ttttacttgc tttaaaaaac ctcccacacc   3360
   tccccctgaa cctgaaacat aaaatgaatg caattgttgt tgttaacttg tttattgcag   3420
   cttataatgg ttacaaataa agcaatagca tcacaaattt cacaaataaa gcattttttt   3480
   cactgcattc tagttgttaa ttaatgcctc gagcgc                              3516
<210> 2
   <211> 5411
   <212> DNA
   <213> Homo sapiens
<220>
   <221> GADD45alpha promoter
   <222> (4) .. (2254)
   <223>
<220>
   <221> GADD45alpha 5' UTR
   <222> (2255)..(2549)
   <223>
<220>
   <221> EGFP gene
   <222> (2550)..(3278)
   <223>
<220>
   <221> GAD45alpha Exon 3
   <222> (3405)..(3642)
   <223>
<220>
   <221> GAD45alpha Intron 3
   <222> (3643)..(4715)
   <223>
<220>
   <221> p53 motif
   <222> (3830)..(3849)
   <223>
<220>
   <221> AP-1 motif
   <222> (3879) .. (3885)
   <223>
<220>
   <221> GADD45alpha Exon 4
   <222> (4716)..(5391)
   <223>
<220>
   <221> GADD45alpha 3' UTR
   <222> (4830)..(5391)
   <223>
<400> 2
   atcagatctt gggtggggca ctttaggact gtggttcatt tgaattggtg taaacaatac    60
   accggttcta ctgtcctaca gcctccattc agatgactga agtcatggga ctttcagcat   120
   agctagctga tgacagtgca tactattttg tcccaaaatc cagttcaagc atggacatac   180
   caataagagc ctaagctctt taaaggcaaa ggaccaggaa ttgtacagtt cttggtatag   240
   aagaagacag gcaaaagtgt ttttgaacta acgttaaatg tgcaatatgt tagaattcat   300
   gcaatgcaca ggactgcagg attctgatat cttatttaac tctcaaattc tattcaactc   360
   aataaacctt gactgtgctt ctactaaatg caggtattgt actaggagct gaggacacca   420
   aactgatgaa gtccttgctg tcaagaaact cacatgattc cctaattctt tgtcagcttg   480
   ctgtgatcac attttcttcc caagaacctc taagaaatgc ctagtggata gaaccttgga   540
   gttccacgga acatattaac aatcgccaaa tgatgactca ggctagattg tgtaattcag   600
   gttttgtctg caaaactgaa aatgcttcgg taacctacct aaatttcaat gttgaggaat   660
   tctttaagaa agacatcaaa tgttaagatt taaggcatag atatgagata catagtcatg   720
   cttaggtgaa ttatgcactg accatgacca tttctttact caaatgttgt ccatggctga   780
   caacacagtg aaaaaatgag tgcaaaatga caactcaaat aaatgaacca gaaaacctat   840
   cacttttctt ttccaccaaa ttaagatcaa gagagctgga gaatattttg tctagagtga   900
   taaaaacata agggtgcaaa acttccaggt acctttgcag aaattacttc tgtgaccttt   960
   ggctgtacag caaccttaat aatgcaagca ctgttttgaa tgcaagcatg tgggagccat  1020
   tttcaccact tttgatgact tcagtaggtt taagaaatgt ttttgctttt attgcataaa  1080
   ccataaaaca aaggaaggga cttttgaact actcagtgag agtctatata ttaaagtttg  1140
   tttttcaaaa atgtgtaact accatttgca gttttaaagg tctgctttcc acctacaagt  1200
   tgccattatc tcaaaggtga aattttagca tatgactaaa aacttcctat agttacagct  1260
   tcatgattca gcatctaaca tcaataattc acagtgagat cataggaggc tctctgtgga  1320
   aggtaacgac atacatacgt taggaaagga agcttagggc atatcgagag cattttgaat  1380
   ttagacttgt gggctgtgtg ggtgtcagat ggttgtctct cagctggtgg gcgtccagaa  1440
   ggatccttgt ttgggcaagg ctctttgaga aaggagaatc tgggttgcca gggattccca  1500
   catgtggtca ccagctcccc acgcagacca gctcacgatt tcccagttac accgggcagg  1560
   tgggaaaccg ttctgctttc tgtggaaaag attctaactt ggttccctgc catccctgaa  1620
   tacaaacggg ttggtttttc ttttttcagc ttccaaccct tgcagctttc caaaaataaa   1680
   tcaaaccagc catcagggca ccgaaataat actactgcta ataagcagct tcgcctagac   1740
   ttagataaac aacacttctg aggtaaactt tgccccggag gtctggagac acttttttaa   1800
   tgtaacctgc ttactaataa ttactagact tcagtgcatt aaccctggaa atagatttta   1860
   atagccaccc cttaaaacaa aagacatgaa aagataataa gaaaaaagtg ccgcaactat   1920
   tatagaaaaa cacttggcag cctgcttcag cccaagctga ggccacctct agcctctgct   1980
   aaagcccccc actcccaatg gtccccgcca accggataag agtgcgcgcg ggacccgcct   2040
   tcccctctcg gcaccgcccc cgcccccgcc ccctcggctc gcctcccgcg tggctcctcc   2100
   cttttccgct cctctcaacc tgactccagg agctggggtc aaattgctgg agcaggctga   2160
   tttgcatagc ccaatggcca agctgcatgc aaatgaggcg gaaggtggtt ggctgagggt   2220
   tggcaggata accccggaga gcggggccct ttgtcctcca gtggctggta ggcagtggct   2280
   gggaggcagc ggcccaatta gtgtcgtgcg gcccgtggcg aggcgaggtc cggggagcga   2340
   gcgagcaagc aaggcgggag gggtggccgg agctgcggcg gctggcacag gaggaggagc   2400
   ccgggcgggc gaggggcggc cggagagcgc cagggcctga gctgccggag cggcgcctgt   2460
   gagtgagtgc agaaagcagg cgcccgcgcg ctagccgtgg caggagcagc ccgcacgccg   2520
   cgctctctcc ctgggcgacc tgcagtttgc aatatggcgc gcctggtgag caagggcgag   2580
   gagctgttca ccggggtggt gcccatcctg gtcgagctgg acggcgacgt aaacggccac   2640
   aagttcagcg tgtccggcga gggcgagggc gatgccacct acggcaagct gaccctgaag   2700
   ttcatctgca ccaccggcaa gctgcccgtg ccctggccca ccctcgtgac caccctgacc   2760
   tacggcgtgc agtgcttcag ccgctacccc gaccacatga agcagcacga cttcttcaag   2820
   tccgccatgc ccgaaggcta cgtccaggag cgcaccatct tcttcaagga cgacggcaac   2880
   tacaagaccc gcgccgaggt gaagttcgag ggcgacaccc tggtgaaccg catcgagctg   2940
   aagggcatcg acttcaagga ggacggcaac atcctggggc acaagctgga gtacaactac   3000
   aacagccaca acgtctatat catggccgac aagcagaaga acggcatcaa ggtgaacttc   3060
   aagatccgcc acaacatcga ggacggcagc gtgcagctcg ccgaccacta ccagcagaac   3120
   acccccatcg gcgacggccc cgtgctgctg cccgacaacc actacctgag cacccagtcc   3180
   gccctgagca aagaccccaa cgagaagcgc gatcacatgg tcctgctgga gttcgtgacc   3240
   gccgccggga tcactctcgg catggacgag ctgtacaagt aaagcggccg cttctgcctg   3300
   tctcggaagg gagggggcga gcgggccggg cggcgacccc cagggacccg ggcagtggtt   3360
   gagggcgccc gcgcttctgc gctcactggc cccgcccgct gcccccagcg accccgataa   3420
   cgtggtgttg tgcctgctgg cggcggacga ggacgacgac agagatgtgg ctctgcagat 3480
   ccacttcacc ctgatccagg cgttttgctg cgagaacgac atcaacatcc tgcgcgtcag 3540
   caacccgggc cggctggcgg agctcctgct cttggagacc gacgctggcc ccgcggcgag 3600
   cgagggcgcc gagcagcccc cggacctgca ctgcgtgctg gtgacggtaa gggactgggg 3660
   gactgcagcc tgcagggtag agccccggaa ggacgggagt cagggctggg ttgcctgatt 3720
   gtggatctgt ggtaggtggg ggtcaggagg gtggctgcct ttgtccgact agagtgtggc 3780
   tggactttca gccgagatgt gctagtttca tcaccaggat tttctgtggt acagaacatg 3840
   tctaagcatg ctggggactg ccagcagcgg aagagatccc tgtgagtcag cagtcagccc 3900
   agctactccc tacctacatc tgcactgcct cccgtgacta attcctttag cagggcagat 3960
   tagataaagc caaatgaatt cctggctcac ccctcattaa ggagtcagct tcattctctg 4020
   ccagtcagag ctaaaaatag aaattgtgta ggagacaaac cttgttaatt ccctagaaat 4080
   acattaagag gatagagtgg aatttttttt ctctgcaatc ttgcattttt ttaatggctc 4140
   tttttttttt tcctgataaa aacctttggt aggtagggaa gttatgtttt caggggtaaa 4200
   tgtgctactt ttgtcttcta aattttgctc ttttttgact ggtctagtca agtgacagcc 4260
   cgattatttt gctactcctt aaaagtacta ttctgtctct tggagtatgg ttgatggcaa 4320
   ttccagttaa ctgctgtgca gctctcatct cattgtgcac acagcatgga aatctttctc 4380
   aaaactgttt cactcaggtc agggtaacaa gtttggtaga gcaaaccggt gaatgatact 4440
   ctcatgcaaa actgaacaga tatgcaaaca tatgtatgtg gttcagcttg ggttgcatgg 4500
   gttcagactt tgcaatgtgt agtttaatag gtaattaccc ttaacgcttt tgcagggaac 4560
   ccaactacct tgaagaaact ttaatttttt tgtgcttcta atttgtctcc atgtcacata 4620
   gccaaaatat agaatgttca agtgttttct cctcaaaagt ataattacta gaatatactg 4680
   gtttttaaaa taagtttatt tttataaatt tgtttccaga atccacattc atctcaatgg 4740
   aaggatcctg ccttaagtca acttatttgt ttttgccggg aaagtcgcta catggatcaa 4800
   tgggttccag tgattaatct ccctgaacgg tgatggcatc tgaatgaaaa taactgaacc 4860
   aaattgcact gaagtttttg aaataccttt gtagttactc aagcagttac tccctacact 4920
   gatgcaagga ttacagaaac tgatgccaag gggctgagtg agttcaacta catgttctgg 4980
   gggcccggag atagatgact ttgcagatgg aaagaggtga aaatgaagaa ggaagctgtg 5040
   ttgaaacaga aaaataagtc aaaaggaaca aaaattacaa agaaccatgc aggaaggaaa 5100
   actatgtatt aatttagaat ggttgagtta cattaaaata aaccaaatat gttaaagttt 5160
   aagtgtgcag ccatagtttg ggtatttttg gtttatatgc cctcaagtaa aagaaaagcc 5220
   gaaagggtta atcatatttg aaaaccatat tttattgtat tttgatgaga tattaaattc 5280
   tcaaagtttt attataaatt ctactaagtt attttatgac atgaaaagtt atttatgcta 5340
   taaatttttt gaaacacaat acctacaata aactggtatg aataattgca tcatttctta 5400
   ttgtgtgctc g 5411
<210> 3
   <211> 5411
   <212> DNA
   <213> Homo sapiens
<220>
   <221> GADD45alpha promoter
   <222> (4)..(2254)
   <223>
<220>
   <221> GADD45alpha 5' UTR
   <222> (2255)..(2549)
   <223>
<220>
   <221> EGFP gene
   <222> (2550)..(3278)
   <223>
<220>
   <221> GADD45alpha Exon 3
   <222> (3405)..(3642)
   <223>
<220>
   <221> GADD45alpha Intron 3
   <222> (3643)..(4715)
   <223>
<220>
   <221> p53 motif
   <222> (3830)..(3849)
   <223>
<220>
   <221> GADD45alpha Exon 4
   <222> (4716)..(5391)
   <223>
<220>
   <221> GADD45alpha 3' UTR
   <222> (4830)..(5391)
   <223>
<400> 3
   atcagatctt gggtggggca ctttaggact gtggttcatt tgaattggtg taaacaatac     60
   accggttcta ctgtcctaca gcctccattc agatgactga agtcatggga ctttcagcat    120
   agctagctga tgacagtgca tactattttg tcccaaaatc cagttcaagc atggacatac    180
   caataagagc ctaagctctt taaaggcaaa ggaccaggaa ttgtacagtt cttggtatag    240
   aagaagacag gcaaaagtgt ttttgaacta acgttaaatg tgcaatatgt tagaattcat    300
   gcaatgcaca ggactgcagg attctgatat cttatttaac tctcaaattc tattcaactc    360
   aataaacctt gactgtgctt ctactaaatg caggtattgt actaggagct gaggacacca    420
   aactgatgaa gtccttgctg tcaagaaact cacatgattc cctaattctt tgtcagcttg    480
   ctgtgatcac attttcttcc caagaacctc taagaaatgc ctagtggata gaaccttgga     540
   gttccacgga acatattaac aatcgccaaa tgatgactca ggctagattg tgtaattcag     600
   gttttgtctg caaaactgaa aatgcttcgg taacctacct aaatttcaat gttgaggaat     660
   tctttaagaa agacatcaaa tgttaagatt taaggcatag atatgagata catagtcatg     720
   cttaggtgaa ttatgcactg accatgacca tttctttact caaatgttgt ccatggctga     780
   caacacagtg aaaaaatgag tgcaaaatga caactcaaat aaatgaacca gaaaacctat     840
   cacttttctt ttccaccaaa ttaagatcaa gagagctgga gaatattttg tctagagtga     900
   taaaaacata agggtgcaaa acttccaggt acctttgcag aaattacttc tgtgaccttt     960
   ggctgtacag caaccttaat aatgcaagca ctgttttgaa tgcaagcatg tgggagccat    1020
   tttcaccact tttgatgact tcagtaggtt taagaaatgt ttttgctttt attgcataaa    1080
   ccataaaaca aaggaaggga cttttgaact actcagtgag agtctatata ttaaagtttg    1140
   tttttcaaaa atgtgtaact accatttgca gttttaaagg tctgctttcc acctacaagt    1200
   tgccattatc tcaaaggtga aattttagca tatgactaaa aacttcctat agttacagct    1260
   tcatgattca gcatctaaca tcaataattc acagtgagat cataggaggc tctctgtgga    1320
   aggtaacgac atacatacgt taggaaagga agcttagggc atatcgagag cattttgaat    1380
   ttagacttgt gggctgtgtg ggtgtcagat ggttgtctct cagctggtgg gcgtccagaa    1440
   ggatccttgt ttgggcaagg ctctttgaga aaggagaatc tgggttgcca gggattccca    1500
   catgtggtca ccagctcccc acgcagacca gctcacgatt tcccagttac accgggcagg    1560
   tgggaaaccg ttctgctttc tgtggaaaag attctaactt ggttccctgc catccctgaa    1620
   tacaaacggg ttggtttttc ttttttcagc ttccaaccct tgcagctttc caaaaataaa     1680
   tcaaaccagc catcagggca ccgaaataat actactgcta ataagcagct tcgcctagac    1740
   ttagataaac aacacttctg aggtaaactt tgccccggag gtctggagac acttttttaa     1800
   tgtaacctgc ttactaataa ttactagact tcagtgcatt aaccctggaa atagatttta    1860
   atagccaccc cttaaaacaa aagacatgaa aagataataa gaaaaaagtg ccgcaactat    1920
   tatagaaaaa cacttggcag cctgcttcag cccaagctga ggccacctct agcctctgct    1980
   aaagcccccc actcccaatg gtccccgcca accggataag agtgcgcgcg ggacccgcct    2040
   tcccctctcg gcaccgcccc cgcccccgcc ccctcggctc gcctcccgcg tggctcctcc    2100
   cttttccgct cctctcaacc tgactccagg agctggggtc aaattgctgg agcaggctga    2160
   tttgcatagc ccaatggcca agctgcatgc aaatgaggcg gaaggtggtt ggctgagggt    2220
   tggcaggata accccggaga gcggggccct ttgtcctcca gtggctggta ggcagtggct    2280
   gggaggcagc ggcccaatta gtgtcgtgcg gcccgtggcg aggcgaggtc cggggagcga 2340
   gcgagcaagc aaggcgggag gggtggccgg agctgcggcg gctggcacag gaggaggagc 2400
   ccgggcgggc gaggggcggc cggagagcgc cagggcctga gctgccggag cggcgcctgt 2460
   gagtgagtgc agaaagcagg cgcccgcgcg ctagccgtgg caggagcagc ccgcacgccg 2520
   cgctctctcc ctgggcgacc tgcagtttgc aatatggcgc gcctggtgag caagggcgag 2580
   gagctgttca ccggggtggt gcccatcctg gtcgagctgg acggcgacgt aaacggccac 2640
   aagttcagcg tgtccggcga gggcgagggc gatgccacct acggcaagct gaccctgaag 2700
   ttcatctgca ccaccggcaa gctgcccgtg ccctggccca ccctcgtgac caccctgacc 2760
   tacggcgtgc agtgcttcag ccgctacccc gaccacatga agcagcacga cttcttcaag 2820
   tccgccatgc ccgaaggcta cgtccaggag cgcaccatct tcttcaagga cgacggcaac 2880
   tacaagaccc gcgccgaggt gaagttcgag ggcgacaccc tggtgaaccg catcgagctg 2940
   aagggcatcg acttcaagga ggacggcaac atcctggggc acaagctgga gtacaactac 3000
   aacagccaca acgtctatat catggccgac aagcagaaga acggcatcaa ggtgaacttc 3060
   aagatccgcc acaacatcga ggacggcagc gtgcagctcg ccgaccacta ccagcagaac 3120
   acccccatcg gcgacggccc cgtgctgctg cccgacaacc actacctgag cacccagtcc 3180
   gccctgagca aagaccccaa cgagaagcgc gatcacatgg tcctgctgga gttcgtgacc 3240
   gccgccggga tcactctcgg catggacgag ctgtacaagt aaagcggccg cgactctaga 3300
   tcataatcag ccataccaca tttgtagagg ttttacttgc tttaaaaaac ctcccacacc 3360
   tccccctgaa cctgaaacat aaaatgaatg caattgttgt tgttatcgcg accccgataa 3420
   cgtggtgttg tgcctgctgg cggcggacga ggacgacgac agagatgtgg ctctgcagat 3480
   ccacttcacc ctgatccagg cgttttgctg cgagaacgac atcaacatcc tgcgcgtcag 3540
   caacccgggc cggctggcgg agctcctgct cttggagacc gacgctggcc ccgcggcgag 3600
   cgagggcgcc gagcagcccc cggacctgca ctgcgtgctg gtgacggtaa gggactgggg 3660
   gactgcagcc tgcagggtag agccccggaa ggacgggagt cagggctggg ttgcctgatt 3720
   gtggatctgt ggtaggtggg ggtcaggagg gtggctgcct ttgtccgact agagtgtggc 3780
   tggactttca gccgagatgt gctagtttca tcaccaggat tttctgtggt acagaacatg 3840
   tctaagcatg ctggggactg ccagcagcgg aagagatccc tgtgagtcag cagtcagccc 3900
   agctactccc tacctacatc tgcactgcct cccgtgacta attcctttag cagggcagat 3960
   tagataaagc caaatgaatt cctggctcac ccctcattaa ggagtcagct tcattctctg 4020
   ccagtcagag ctaaaaatag aaattgtgta ggagacaaac cttgttaatt ccctagaaat 4080
   acattaagag gatagagtgg aatttttttt ctctgcaatc ttgcattttt ttaatggctc 4140
   tttttttttt tcctgataaa aacctttggt aggtagggaa gttatgtttt caggggtaaa 4200
   tgtgctactt ttgtcttcta aattttgctc ttttttgact ggtctagtca agtgacagcc 4260
   cgattatttt gctactcctt aaaagtacta ttctgtctct tggagtatgg ttgatggcaa 4320
   ttccagttaa ctgctgtgca gctctcatct cattgtgcac acagcatgga aatctttctc 4380
   aaaactgttt cactcaggtc agggtaacaa gtttggtaga gcaaaccggt gaatgatact 4440
   ctcatgcaaa actgaacaga tatgcaaaca tatgtatgtg gttcagcttg ggttgcatgg 4500
   gttcagactt tgcaatgtgt agtttaatag gtaattaccc ttaacgcttt tgcagggaac 4560
   ccaactacct tgaagaaact ttaatttttt tgtgcttcta atttgtctcc atgtcacata 4620
   gccaaaatat agaatgttca agtgttttct cctcaaaagt ataattacta gaatatactg 4680
   gtttttaaaa taagtttatt tttataaatt tgtttccaga atccacattc atctcaatgg 4740
   aaggatcctg ccttaagtca acttatttgt ttttgccggg aaagtcgcta catggatcaa 4800
   tgggttccag tgattaatct ccctgaacgg tgatggcatc tgaatgaaaa taactgaacc 4860
   aaattgcact gaagtttttg aaataccttt gtagttactc aagcagttac tccctacact 4920
   gatgcaagga ttacagaaac tgatgccaag gggctgagtg agttcaacta catgttctgg 4980
   gggcccggag atagatgact ttgcagatgg aaagaggtga aaatgaagaa ggaagctgtg 5040
   ttgaaacaga aaaataagtc aaaaggaaca aaaattacaa agaaccatgc aggaaggaaa 5100
   actatgtatt aatttagaat ggttgagtta cattaaaata aaccaaatat gttaaagttt 5160
   aagtgtgcag ccatagtttg ggtatttttg gtttatatgc cctcaagtaa aagaaaagcc 5220
   gaaagggtta atcatatttg aaaaccatat tttattgtat tttgatgaga tattaaattc 5280
   tcaaagtttt attataaatt ctactaagtt attttatgac atgaaaagtt atttatgcta 5340
   taaatttttt gaaacacaat acctacaata aactggtatg aataattgca tcatttctta 5400
   ttgtgtgctc g 5411
<210> 4
   <211> 5413
   <212> DNA
   <213> Homo sapiens
<220>
   <221> GADD45alpha promoter
   <222> (4) .. (2254)
   <223>
<220>
   <221> GADD45alpha 5' UTR
   <222> (2255)..(2549)
   <223>
<220>
   <221> EGFP gene
   <222> (2550)..(3278)
   <223>
<220>
   <221> SV40 polyA
   <222> (3432)..(3482)
   <223>
<220>
   <221> GADD45alpha Exon 3
   <222> (3502)..(3642)
   <223>
<220>
   <221> GADD45alpha Intron 3
   <222> (3643)..(4715)
   <223>
<220>
   <221> p53 motif
   <222> (3830)..(3849)
   <223>
<220>
   <221> AP-1 motif
   <222> (3879) .. (3885)
   <223>
<220>
   <221> GADD45alpha Exon 4
   <222> (4716)..(5391)
   <223>
<220>
   <221> GADD45alpha 3' UTR
   <222> (4830)..(5391)
   <223>
<400> 4
   atcagatctt gggtggggca ctttaggact gtggttcatt tgaattggtg taaacaatac    60
   accggttcta ctgtcctaca gcctccattc agatgactga agtcatggga ctttcagcat   120
   agctagctga tgacagtgca tactattttg tcccaaaatc cagttcaagc atggacatac   180
   caataagagc ctaagctctt taaaggcaaa ggaccaggaa ttgtacagtt cttggtatag   240
   aagaagacag gcaaaagtgt ttttgaacta acgttaaatg tgcaatatgt tagaattcat   300
   gcaatgcaca ggactgcagg attctgatat cttatttaac tctcaaattc tattcaactc   360
   aataaacctt gactgtgctt ctactaaatg caggtattgt actaggagct gaggacacca   420
   aactgatgaa gtccttgctg tcaagaaact cacatgattc cctaattctt tgtcagcttg   480
   ctgtgatcac attttcttcc caagaacctc taagaaatgc ctagtggata gaaccttgga    540
   gttccacgga acatattaac aatcgccaaa tgatgactca ggctagattg tgtaattcag    600
   gttttgtctg caaaactgaa aatgcttcgg taacctacct aaatttcaat gttgaggaat    660
   tctttaagaa agacatcaaa tgttaagatt taaggcatag atatgagata catagtcatg    720
   cttaggtgaa ttatgcactg accatgacca tttctttact caaatgttgt ccatggctga    780
   caacacagtg aaaaaatgag tgcaaaatga caactcaaat aaatgaacca gaaaacctat    840
   cacttttctt ttccaccaaa ttaagatcaa gagagctgga gaatattttg tctagagtga    900
   taaaaacata agggtgcaaa acttccaggt acctttgcag aaattacttc tgtgaccttt    960
   ggctgtacag caaccttaat aatgcaagca ctgttttgaa tgcaagcatg tgggagccat   1020
   tttcaccact tttgatgact tcagtaggtt taagaaatgt ttttgctttt attgcataaa   1080
   ccataaaaca aaggaaggga cttttgaact actcagtgag agtctatata ttaaagtttg   1140
   tttttcaaaa atgtgtaact accatttgca gttttaaagg tctgctttcc acctacaagt   1200
   tgccattatc tcaaaggtga aattttagca tatgactaaa aacttcctat agttacagct   1260
   tcatgattca gcatctaaca tcaataattc acagtgagat cataggaggc tctctgtgga   1320
   aggtaacgac atacatacgt taggaaagga agcttagggc atatcgagag cattttgaat   1380
   ttagacttgt gggctgtgtg ggtgtcagat ggttgtctct cagctggtgg gcgtccagaa   1440
   ggatccttgt ttgggcaagg ctctttgaga aaggagaatc tgggttgcca gggattccca   1500
   catgtggtca ccagctcccc acgcagacca gctcacgatt tcccagttac accgggcagg   1560
   tgggaaaccg ttctgctttc tgtggaaaag attctaactt ggttccctgc catccctgaa   1620
   tacaaacggg ttggtttttc ttttttcagc ttccaaccct tgcagctttc caaaaataaa   1680
   tcaaaccagc catcagggca ccgaaataat actactgcta ataagcagct tcgcctagac   1740
   ttagataaac aacacttctg aggtaaactt tgccccggag gtctggagac acttttttaa    1800
   tgtaacctgc ttactaataa ttactagact tcagtgcatt aaccctggaa atagatttta   1860
   atagccaccc cttaaaacaa aagacatgaa aagataataa gaaaaaagtg ccgcaactat   1920
   tatagaaaaa cacttggcag cctgcttcag cccaagctga ggccacctct agcctctgct   1980
   aaagcccccc actcccaatg gtccccgcca accggataag agtgcgcgcg ggacccgcct   2040
   tcccctctcg gcaccgcccc cgcccccgcc ccctcggctc gcctcccgcg tggctcctcc    2100
   cttttccgct cctctcaacc tgactccagg agctggggtc aaattgctgg agcaggctga   2160
   tttgcatagc ccaatggcca agctgcatgc aaatgaggcg gaaggtggtt ggctgagggt   2220
   tggcaggata accccggaga gcggggccct ttgtcctcca gtggctggta ggcagtggct   2280
   gggaggcagc ggcccaatta gtgtcgtgcg gcccgtggcg aggcgaggtc cggggagcga 2340
   gcgagcaagc aaggcgggag gggtggccgg agctgcggcg gctggcacag gaggaggagc 2400
   ccgggcgggc gaggggcggc cggagagcgc cagggcctga gctgccggag cggcgcctgt 2460
   gagtgagtgc agaaagcagg cgcccgcgcg ctagccgtgg caggagcagc ccgcacgccg 2520
   cgctctctcc ctgggcgacc tgcagtttgc aatatggcgc gcctggtgag caagggcgag 2580
   gagctgttca ccggggtggt gcccatcctg gtcgagctgg acggcgacgt aaacggccac 2640
   aagttcagcg tgtccggcga gggcgagggc gatgccacct acggcaagct gaccctgaag 2700
   ttcatctgca ccaccggcaa gctgcccgtg ccctggccca ccctcgtgac caccctgacc 2760
   tacggcgtgc agtgcttcag ccgctacccc gaccacatga agcagcacga cttcttcaag 2820
   tccgccatgc ccgaaggcta cgtccaggag cgcaccatct tcttcaagga cgacggcaac 2880
   tacaagaccc gcgccgaggt gaagttcgag ggcgacaccc tggtgaaccg catcgagctg 2940
   aagggcatcg acttcaagga ggacggcaac atcctggggc acaagctgga gtacaactac 3000
   aacagccaca acgtctatat catggccgac aagcagaaga acggcatcaa ggtgaacttc 3060
   aagatccgcc acaacatcga ggacggcagc gtgcagctcg ccgaccacta ccagcagaac 3120
   acccccatcg gcgacggccc cgtgctgctg cccgacaacc actacctgag cacccagtcc 3180
   gccctgagca aagaccccaa cgagaagcgc gatcacatgg tcctgctgga gttcgtgacc 3240
   gccgccggga tcactctcgg catggacgag ctgtacaagt aaagcggccg cgactctaga 3300
   tcataatcag ccataccaca tttgtagagg ttttacttgc tttaaaaaac ctcccacacc 3360
   tccccctgaa cctgaaacat aaaatgaatg caattgttgt tgttaacttg tttattgcag 3420
   cttataatgg ttacaaataa agcaatagca tcacaaattt cacaaataaa gcattttttt 3480
   cactgcattc tagttgttaa ttaattgctg cgagaacgac atcaacatcc tgcgcgtcag 3540
   caacccgggc cggctggcgg agctcctgct cttggagacc gacgctggcc ccgcggcgag 3600
   cgagggcgcc gagcagcccc cggacctgca ctgcgtgctg gtgacggtaa gggactgggg 3660
   gactgcagcc tgcagggtag agccccggaa ggacgggagt cagggctggg ttgcctgatt 3720
   gtggatctgt ggtaggtggg ggtcaggagg gtggctgcct ttgtccgact agagtgtggc 3780
   tggactttca gccgagatgt gctagtttca tcaccaggat tttctgtggt acagaacatg 3840
   tctaagcatg ctggggactg ccagcagcgg aagagatccc tgtgagtcag cagtcagccc 3900
   agctactccc tacctacatc tgcactgcct cccgtgacta attcctttag cagggcagat 3960
   tagataaagc caaatgaatt cctggctcac ccctcattaa ggagtcagct tcattctctg 4020
   ccagtcagag ctaaaaatag aaattgtgta ggagacaaac cttgttaatt ccctagaaat    4080
   acattaagag gatagagtgg aatttttttt ctctgcaatc ttgcattttt ttaatggctc    4140
   tttttttttt tcctgataaa aacctttggt aggtagggaa gttatgtttt caggggtaaa    4200
   tgtgctactt ttgtcttcta aattttgctc ttttttgact ggtctagtca agtgacagcc    4260
   cgattatttt gctactcctt aaaagtacta ttctgtctct tggagtatgg ttgatggcaa    4320
   ttccagttaa ctgctgtgca gctctcatct cattgtgcac acagcatgga aatctttctc    4380
   aaaactgttt cactcaggtc agggtaacaa gtttggtaga gcaaaccggt gaatgatact    4440
   ctcatgcaaa actgaacaga tatgcaaaca tatgtatgtg gttcagcttg ggttgcatgg    4500
   gttcagactt tgcaatgtgt agtttaatag gtaattaccc ttaacgcttt tgcagggaac    4560
   ccaactacct tgaagaaact ttaatttttt tgtgcttcta atttgtctcc atgtcacata    4620
   gccaaaatat agaatgttca agtgttttct cctcaaaagt ataattacta gaatatactg    4680
   gtttttaaaa taagtttatt tttataaatt tgtttccaga atccacattc atctcaatgg    4740
   aaggatcctg ccttaagtca acttatttgt ttttgccggg aaagtcgcta catggatcaa    4800
   tgggttccag tgattaatct ccctgaacgg tgatggcatc tgaatgaaaa taactgaacc    4860
   aaattgcact gaagtttttg aaataccttt gtagttactc aagcagttac tccctacact    4920
   gatgcaagga ttacagaaac tgatgccaag gggctgagtg agttcaacta catgttctgg    4980
   gggcccggag atagatgact ttgcagatgg aaagaggtga aaatgaagaa ggaagctgtg    5040
   ttgaaacaga aaaataagtc aaaaggaaca aaaattacaa agaaccatgc aggaaggaaa    5100
   actatgtatt aatttagaat ggttgagtta cattaaaata aaccaaatat gttaaagttt    5160
   aagtgtgcag ccatagtttg ggtatttttg gtttatatgc cctcaagtaa aagaaaagcc    5220
   gaaagggtta atcatatttg aaaaccatat tttattgtat tttgatgaga tattaaattc    5280
   tcaaagtttt attataaatt ctactaagtt attttatgac atgaaaagtt atttatgcta    5340
   taaatttttt gaaacacaat acctacaata aactggtatg aataattgca tcatttctta    5400
   ttgtgtgctc gag                                                       5413
<210> 5
   <211> 3871
   <212> DNA
   <213> Homo sapiens
<220>
   <221> GADD45alpha promoter
   <222> (4)..(2254)
   <223>
<220>
   <221> GADD45alpha 5' UTR
   <222> (2255) .. (2549)
   <223>
<220>
   <221> EGFP gene
   <222> (2550)..(3278)
   <223>
<220>
   <221> GADD45alpha 3' UTR
   <222> (3288) .. (3849)
   <223>
<400> 5
   atcagatctt gggtggggca ctttaggact gtggttcatt tgaattggtg taaacaatac      60
   accggttcta ctgtcctaca gcctccattc agatgactga agtcatggga ctttcagcat     120
   agctagctga tgacagtgca tactattttg tcccaaaatc cagttcaagc atggacatac     180
   caataagagc ctaagctctt taaaggcaaa ggaccaggaa ttgtacagtt cttggtatag     240
   aagaagacag gcaaaagtgt ttttgaacta acgttaaatg tgcaatatgt tagaattcat     300
   gcaatgcaca ggactgcagg attctgatat cttatttaac tctcaaattc tattcaactc     360
   aataaacctt gactgtgctt ctactaaatg caggtattgt actaggagct gaggacacca     420
   aactgatgaa gtccttgctg tcaagaaact cacatgattc cctaattctt tgtcagcttg     480
   ctgtgatcac attttcttcc caagaacctc taagaaatgc ctagtggata gaaccttgga     540
   gttccacgga acatattaac aatcgccaaa tgatgactca ggctagattg tgtaattcag    600
   gttttgtctg caaaactgaa aatgcttcgg taacctacct aaatttcaat gttgaggaat    660
   tctttaagaa agacatcaaa tgttaagatt taaggcatag atatgagata catagtcatg    720
   cttaggtgaa ttatgcactg accatgacca tttctttact caaatgttgt ccatggctga    780
   caacacagtg aaaaaatgag tgcaaaatga caactcaaat aaatgaacca gaaaacctat    840
   cacttttctt ttccaccaaa ttaagatcaa gagagctgga gaatattttg tctagagtga    900
   taaaaacata agggtgcaaa acttccaggt acctttgcag aaattacttc tgtgaccttt    960
   ggctgtacag caaccttaat aatgcaagca ctgttttgaa tgcaagcatg tgggagccat   1020
   tttcaccact tttgatgact tcagtaggtt taagaaatgt tttgcttttt attgcataaa   1080
   ccataaaaca aaggaaggga cttttgaact actcagtgag agtctatata ttaaagtttg   1140
   tttttcaaaa atgtgtaact accatttgca gttttaaagg tctgctttcc acctacaagt   1200
   tgccattatc tcaaaggtga aattttagca tatgactaaa aacttcctat agttacagct   1260
   tcatgattca gcatctaaca tcaataattc acagtgagat cataggaggc tctctgtgga   1320
   aggtaacgac atacatacgt taggaaagga agcttagggc atatcgagag cattttgaat   1380
   ttagacttgt gggctgtgtg ggtgtcagat ggttgtctct cagctggtgg gcgtccagaa   1440
   ggatccttgt ttgggcaagg ctctttgaga aaggagaatc tgggttgcca gggattccca   1500
   catgtggtca ccagctcccc acgcagacca gctcacgatt tcccagttac accgggcagg   1560
   tgggaaaccg ttctgctttc tgtggaaaag attctaactt ggttccctgc catccctgaa   1620
   tacaaacggg ttggtttttc ttttttcagc ttccaaccct tgcagctttc caaaaataaa   1680
   tcaaaccagc catcagggca ccgaaataat actactgcta ataagcagct tcgcctagac   1740
   ttagataaac aacacttctg aggtaaactt tgccccggag gtctggagac acttttttaa    1800
   tgtaacctgc ttactaataa ttactagact tcagtgcatt aaccctggaa atagatttta   1860
   atagccaccc cttaaaacaa aagacatgaa aagataataa gaaaaaagtg ccgcaactat   1920
   tatagaaaaa cacttggcag cctgcttcag cccaagctga ggccacctct agcctctgct   1980
   aaagcccccc actcccaatg gtccccgcca accggataag agtgcgcgcg ggacccgcct   2040
   tcccctctcg gcaccgcccc cgcccccgcc ccctcggctc gcctcccgcg tggctcctcc   2100
   cttttccgct cctctcaacc tgactccagg agctggggtc aaattgctgg agcaggctga   2160
   tttgcatagc ccaatggcca agctgcatgc aaatgaggcg gaaggtggtt ggctgagggt   2220
   tggcaggata accccggaga gcggggccct ttgtcctcca gtggctggta ggcagtggct   2280
   gggaggcagc ggcccaatta gtgtcgtgcg gcccgtggcg aggcgaggtc cggggagcga   2340
   gcgagcaagc aaggcgggag gggtggccgg agctgcggcg gctggcacag gaggaggagc    2400
   ccgggcgggc gaggggcggc cggagagcgc cagggcctga gctgccggag cggcgcctgt    2460
   gagtgagtgc agaaagcagg cgcccgcgcg ctagccgtgg caggagcagc ccgcacgccg    2520
   cgctctctcc ctgggcgacc tgcagtttgc aatatggcgc gcctggtgag caagggcgag    2580
   gagctgttca ccggggtggt gcccatcctg gtcgagctgg acggcgacgt aaacggccac    2640
   aagttcagcg tgtccggcga gggcgagggc gatgccacct acggcaagct gaccctgaag    2700
   ttcatctgca ccaccggcaa gctgcccgtg ccctggccca ccctcgtgac caccctgacc    2760
   tacggcgtgc agtgcttcag ccgctacccc gaccacatga agcagcacga cttcttcaag    2820
   tccgccatgc ccgaaggcta cgtccaggag cgcaccatct tcttcaagga cgacggcaac    2880
   tacaagaccc gcgccgaggt gaagttcgag ggcgacaccc tggtgaaccg catcgagctg    2940
   aagggcatcg acttcaagga ggacggcaac atcctggggc acaagctgga gtacaactac    3000
   aacagccaca acgtctatat catggccgac aagcagaaga acggcatcaa ggtgaacttc    3060
   aagatccgcc acaacatcga ggacggcagc gtgcagctcg ccgaccacta ccagcagaac    3120
   acccccatcg gcgacggccc cgtgctgctg cccgacaacc actacctgag cacccagtcc    3180
   gccctgagca aagaccccaa cgagaagcgc gatcacatgg tcctgctgga gttcgtgacc    3240
   gccgccggga tcactctcgg catggacgag ctgtacaagt aaagcggccg ctggcatctg    3300
   aatgaaaata actgaaccaa attgcactga agtttttgaa atacctttgt agttactcaa    3360
   gcagttactc cctacactga tgcaaggatt acagaaactg atgccaaggg gctgagtgag    3420
   ttcaactaca tgttctgggg gcccggagat agatgacttt gcagatggaa agaggtgaaa    3480
   atgaagaagg aagctgtgtt gaaacagaaa aataagtcaa aaggaacaaa aattacaaag    3540
   aaccatgcag gaaggaaaac tatgtattaa tttagaatgg ttgagttaca ttaaaataaa    3600
   ccaaatatgt taaagtttaa gtgtgcagcc atagtttggg tatttttggt ttatatgccc    3660
   tcaagtaaaa gaaaagccga aagggttaat catatttgaa aaccatattt tattgtattt    3720
   tgatgagata ttaaattctc aaagttttat tataaattct actaagttat tttatgacat    3780
   gaaaagttat ttatgctata aattttttga aacacaatac ctacaataaa ctggtatgaa    3840
   taattgcatc atttcttatt gtgtgctcga g                                    3871

## Description

The present invention relates to methods for detecting agents that cause or potentiate DNA damage, and to molecules and transfected cell lines that may be employed in such methods. In particular, the invention relates to biosensors for detecting DNA damage in human cell cultures.

DNA damage is induced by a variety of agents such as ultraviolet light, X rays, free radicals, methylating agents and other mutagenic compounds. DNA damage can also be caused indirectly either by agents that affect enzymes and proteins which interact with DNA (including polymerases and topoisomerases) or by promutagens (agents that can be metabolised to become mutagenic). Any of these agents may cause damage to the DNA that comprises the genetic code of an organism and cause mutations in genes. In animals, such mutations can lead to carcinogenesis or may damage the gametes to give rise to congenital defects in offspring. Such DNA damaging agents can be collectively known as genotoxins.

These DNA damaging agents may chemically modify the nucleotides that comprise DNA and may also break the phosphodiester bonds that link the nucleotides or disrupt association between bases (T-A or C-G). To counter the effect of these DNA damaging agents cells have evolved a number of mechanisms. For example, the SOS response in E. *coli* is a well-characterised cellular response induced by DNA damage in which a series of proteins are expressed, including DNA repair enzymes, which repair the damaged DNA. In mammalians, nucleotide excision repair and base excision repair mechanisms play a prominent role in DNA damage repair, and are the primary mechanism for removal of bulky DNA adducts and modified bases.

There are numerous circumstances when it is important to identify what agents may cause or potentiate DNA damage. It is particularly important to detect agents that cause DNA damage when assessing whether it is safe to expose a person to these agents. For instance, a method of detecting these agents may be used as a genotoxicity assay for screening compounds that are candidate medicaments, food additives or cosmetics to assess whether or not the compound of interest induces DNA damage. Alternatively, methods of detecting DNA damaging agents may be used to monitor for contamination of water supplies with pollutants that contain mutagenic compounds.

Various methods, such as the Ames Test, the *in vitro* micronucleus test and the mouse lymphoma assay (MLA), for determining the toxicity of an agent are known but are unsatisfactory for a number of reasons. For instance, incubation of samples can take many weeks, when it is often desirable to obtain genotoxic data in a shorter time frame. Furthermore, many known methods of detecting DNA damage (including the Ames Test and related methods) assay lasting DNA damage, as an endpoint, either in the form of mis-repaired DNA (mutations and recombinations) or unrepaired damage in the form of fragmented DNA. However, most DNA damage is repaired before such an endpoint can be measured and lasting DNA damage only occurs if the conditions are so severe that the repair mechanisms have been saturated.

An improved genotoxic test is disclosed in WO 98/44149, which concerns recombinant DNA molecules comprising a *Saccharomyces cerevisiaie* regulatory element that activates gene expression in response to DNA damage operatively linked to a DNA sequence that encodes a light emitting reporter protein, such as Green Fluorescent Protein (GFP). Such DNA molecules may be used to transform a yeast cell for use in a genotoxic test for detecting for the presence of an agent that causes or potentiates DNA damage. The cells may be subjected to an agent and the expression of the light emitting reporter protein (GFP) from the cell indicates that the agent causes DNA damage. The genotoxic tests described in WO 98/44149 detect the induction of repair activity that can prevent an endpoint being reached. The method described in WO 98/44149 may therefore be used to detect for the presence of DNA damaging agents.

US 6,344,324 discloses a recombinant DNA molecule comprising the regulatory element of the hamster *GADD153* upstream promoter region that activates gene expression in response to a wide range of cellular stress conditions, linked to a DNA sequence that encodes GFP. This reporter system is carried out in a human head and neck squamous-cell carcinoma cell line. However, problems associated with this reporter system are that it requires at least a four day treatment period at test agent concentrations that result in less than 10% cell survival, followed by analysis of fluorescence by flow cytometry. In addition, the biological relevance of any gene induction when tested with agents at this level of toxicity is debatable. Furthermore, this development does not disclose a means of specifically monitoring for the presence of agents that may cause or potentiate DNA damage, and the mechanism of *GADD153* induction remains unclear. Hence, this system is of very limited use as a human DNA damage biosensor.

Therefore, it is an aim of embodiments of the present invention to address problems associated with the prior art, and to provide an improved biosensor for detecting DNA damage in human cell cultures.

According to a first aspect of the present invention, there is provided an expression cassette comprising a DNA sequence encoding a reporter protein, which DNA sequence is operatively linked to a human GADD45α gene promoter and a human GADD45α gene regulatory element arranged to activate expression of the DNA sequence in response to DNA damage.

By the term "regulatory element", we mean a DNA sequence that regulates the transcription of a gene with which it is associated, i.e. the DNA sequence encoding the reporter protein.

By the term "operatively linked", we mean that the regulatory element is able to induce the expression of the reporter protein.

According to a second aspect of the invention, there is provided a recombinant vector comprising an expression cassette according to the first aspect.

According to a third aspect of the invention, there is provided a cell containing a recombinant vector in accordance with the second aspect of the present invention.

According to a fourth aspect of the present invention, there is provided a method of detecting for the presence of an agent that causes or potentiates DNA damage comprising subjecting a cell in accordance with the third aspect of the present invention to an agent; and monitoring the expression of the reporter protein from the cell.

The method of the fourth aspect of the invention represents a novel cost-effective genotoxicity screen that may be used to provide a pre-regulatory screening assay for use by the pharmaceutical industry and in other applications where significant numbers of agents or compounds need to be tested. It provides a higher throughput and a lower compound consumption than existing *in vitro* and *in vivo* mammalian genotoxicity assays, and is sensitive to a broad spectrum of mutagens.

The method of the fourth aspect of the invention is suitable for assessing whether or not an agent may cause DNA damage. It is particularly useful for detecting agents that cause DNA damage when assessing whether it is safe to expose a person to DNA damaging agents. For instance, the method may be used as a genotoxicity assay for screening whether or not known agents, such as candidate medicaments, pharmaceutical and industrial chemicals, pesticides, fungicides, foodstuffs or cosmetics, induce DNA damage. Alternatively, the method of the invention may be used to monitor for contamination of water supplies, leachates and effluents with pollutants containing DNA damaging agents.

The method of the fourth aspect of the invention may also be used for assessing whether an agent may potentiate DNA damage. For example, certain agents can cause accumulation of DNA damage by inhibiting DNA repair (for instance by preventing expression or function of a repair protein) without directly inflicting DNA damage.

Surprisingly, the use of a human GADD45α gene regulatory element in addition to the human GADD45α gene promoter in the expression cassette according to the first aspect of the invention radically enhances the response of the cassette to genotoxic stress and, hence, DNA damage in the cell according to the third aspect. Advantageously, the cassette can be analysed for expression of the reporter protein within or after only 24 hours simply by assaying for the protein in a test culture. The cells may be subjected to the test agent or compound, and expression of the reporter protein in the cell indicates whether the test agent causes DNA damage.

The inventors have found that DNA encoding a human GADD45a gene promoter and a human GADD45α gene regulatory element may be operatively linked a reporter protein to form a cassette according to the first aspect of the invention and then advantageously used in a genotoxic test according to the fourth aspect of the invention. Such cassettes may comprise the whole of the GADD45α gene (including coding sequences) provided that it is operatively linked to DNA encoding a light emitting reporter. For instance cassettes may be made according to the first aspect of the invention comprising the whole of, or substantially all of, the GADD45α gene (comprising regulatory elements and promoter) with DNA encoding a reporter inserted 3' of the GADD45α promoter (e.g. within the GADD45α coding sequence or at the 3' of the coding sequence). end of the arranged to activate expression of the DNA sequence in response to DNA damage.

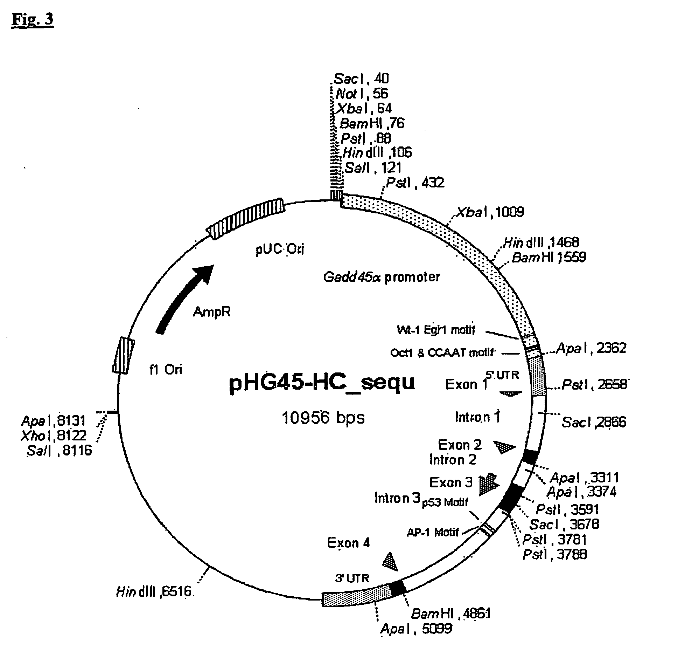

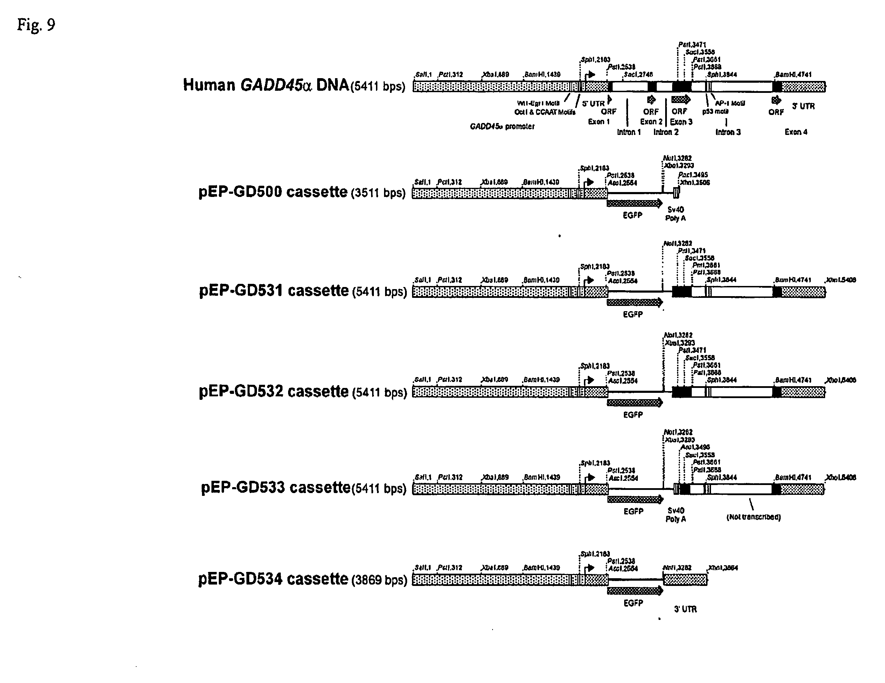

Preferably, the human GADD45α gene promoter sequence induces RNA polymerase to bind to the DNA molecule and start transcribing the DNA encoding the reporter protein. It is preferred that the promoter sequence comprises the human GADD45α gene promoter sequence and the 5' untranslated region. The promoter sequence may be obtained from the pHG45-HC plasmid, which is illustrated in Figure 3. The promoter sequence can be seen in each of the expression cassettes in accordance with the invention in Figure 9, and the nucleotide sequence of the GADD45α gene promoter is shown as bases 4-2254 in SEQ ID No.s 1, 2, 3, 4 & 5 in the sequence listing. It will be appreciated that the promoter may comprise each of the bases 4 -2254 or alternatively may be a functional derivative or functional fragment thereof. Functional derivatives and functional fragments may be readily identified be assessing whether or not transcriptase will bind to a putative promoter region and will then lead to the transcription of the marker protein. Alternatively such functional derivatives and fragments may be examined by conducting mutagenesis on the GADD45α promoter, when in natural association with the GADD45α gene, and assessing whether or not GADD45α expression may occur.

The regulatory element in the expression cassette according to the invention may comprise sequences downstream of the GADD45α gene promoter sequence. The regulatory element may comprise functional DNA sequences such as those encoding translation initiation sequences for ribosome binding or DNA sequences that bind transcription factors which promote gene expression following DNA damage. The regulatory element in the expression cassette according to the invention may comprise at least one exon of the GADD45α gene. For example, the regulatory element may comprise Exon 1, Exon 2, Exon 3, and/or Exon 4 of the GADD45α gene, or at least a region thereof, or any combination thereof. Hence, the regulatory element may comprise any combination of the four exons of the GADD45α gene, or at least a region thereof.

In a preferred embodiment, the regulatory element comprises at least a region of Exon 1 of the GADD45α gene, and preferably at least a region of Exon 3 of the GADD45α gene, and more preferably, at least a region of Exon 4 of the GADD45α gene. It is especially preferred that the regulatory element comprises all of Exon 1 of the GADD45α gene, and preferably at least a region of Exon 3 of the GADD45α gene, and more preferably, all of Exon 4 of the GADD45α gene.

Preferred regulatory elements are illustrated in the each of the expression cassettes in accordance with the invention in Figure 9. The nucleotide sequence of Exon 3 of the GADD45α gene is shown as bases 3405-3642 in SEQ ID No.s 2, 3 & 4 in the sequence listing. The nucleotide sequence of the preferred region of Exon 3 of the GADD45α gene is shown as bases 3503-3642 in SEQ ID No.4. The nucleotide sequence of Exon 4 of the GADD45α gene is shown as bases 4716-5391 in SEQ ID No.s 2, 3 & 4 in the sequence listing.

Alternatively, or additionally, the regulatory element may comprise a non-coding DNA sequence, for example, at least one intron of the GADD45α gene. For example, the regulatory element may comprise Intron 1, Intron 2, and/or Intron 3 of the GADD45α gene, or at least a region thereof, or any combination thereof. Hence, the regulatory element may comprise any combination of the three introns of the GADD45α gene, or at least a region thereof.

In a preferred embodiment, the regulatory element in the expression cassette according to the invention comprises at least a region of Intron 3 of the GADD45α gene. Intron 3 of GADD45α is illustrated in Figure 9, and the nucleotide sequence of Intron 3 of the GADD45α gene is shown as bases 3643-4715 in SEQ ID No.s 2, 3 & 4 in the sequence listing.

In a preferred embodiment, the expression cassette in accordance with the invention comprises the promoter sequence of the *GADD45*α gene and also gene regulatory elements found within Intron 3 of the genomic *GADD45*α gene sequence itself. While the inventors do not wish to be bound by any hypothesis, they believe that Intron 3 of the GADD45α gene, contains a putative p53 binding motif, and that it is this p53 motif which surprisingly enhances the response of the expression cassette to genotoxic stress. The putative p53 binding motif is shown as nucleotide bases 3830-3849 of SEQ ID No.s 2, 3 and 4.

The inventors also believe that Intron 3 of the *GADD45*α gene may contain a putative TRE motif, which may encode a AP-1 binding site. The putative TRE motif is shown as nucleotide bases 3879-3885 of SEQ ID No.s 2, 3 and 4. Hence, while the inventors do not wish to be bound by any hypothesis, they postulate that this putative AP-1 binding site may also contribute to the improved response to genotoxic agents.

It is preferred that the expression cassette comprises at least the p53 binding motif and/or the AP-1 binding motif from Intron 3 of the *GADD45*α gene.

The regulatory element may comprise a 3' untranslated (UTR) region of the *GADD45*α gene, the nucleotide sequence of which is shown as bases 4830-5391 in SEQ ID No.s 2, 3, and 4. While the inventors do not wish to be bound by any hypothesis, they believe that this 3' UTR may be involved with stabilisation of mRNA cassette, and hence, may be surprisingly important when used with the rest of the regulatory element, such as Intron 3.

By the term "reporter protein", we mean a protein which when expressed in response to the regulatory element of the DNA molecule of the invention, is detectable by means of a suitable assay procedure. Preferably, the reporter protein comprises a light emitting reporter protein. The DNA sequence that encodes a light emitting reporter protein may code for any light emitting protein, for example Luciferase or Green Fluorescent Protein. However, it is preferred that the DNA sequence codes for a protein that is substantially fluorescent.

Preferred DNA sequences that encode a light emitting reporter protein code for Green Fluorescent Protein (GFP), and light emitting derivatives thereof. GFP is from the jelly fish *Aequorea victoria* and is able to absorb blue light and re-emits an easily detectable green light and is thus suitable as a reporter protein. GFP may be advantageously used as a reporter protein because its measurement is simple and reagent free and the protein is non-toxic.

Derivatives of GFP include DNA sequences encoding for polypeptide analogues or polypeptide fragments of GFP, which are able to emit light. Many of these derivatives absorb and re-emit light at wavelengths different to GFP found endogenously in *Aequorea victoria*.

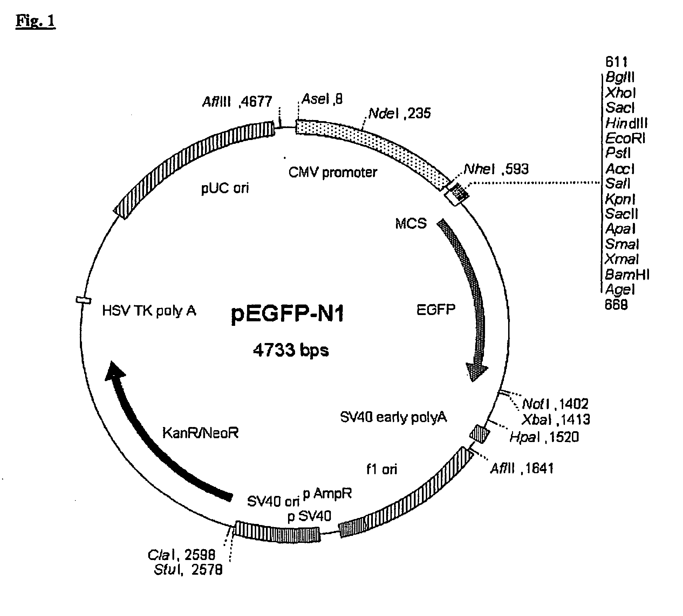

For instance, preferred expression cassettes according to the first aspect of the invention comprise a DNA sequence that encodes the human enhanced GFP (hEGFP), also known as GFPmut1 variant of GFP. This consists of the GFP wild type containing a double amino acid substitution of Phe-64 to Leu, and Ser-65 to Thr. The coding sequence of the hEGFP gene also contains more than 190 silent base changes, which correspond to human codon usage preferences relative to the native *Aequorea victoria* sequence. This is particularly advantageous for use in a human DNA damage reporter system. The hEGFP sequence may be obtained from the pEGFP-N1 plasmid (e.g. obtained from Clontech), which is shown in Figure 1. The hEGFP sequence can be seen in each of the expression cassettes in accordance with the invention in Figure 9, and the nucleotide sequence of the hEGFP is shown as bases 2550-3278 in SEQ ID No.s 1, 2, 3, 4 & 5 in the sequence listing.

GFP produces a good quantum yield of fluorescence and matches the output of argon ion lasers used in fluorescence activated cell sorters. Cells according to the third aspect of the invention, which contain DNA molecules coding hEGFP, may be used according to the method of the fourth aspect of the invention.

Hence, preferred expression cassettes according to the invention comprise a human GADD45α gene regulatory element and human GADD45α gene promoter operatively linked to a DNA sequence encoding a GFP, or light emitting derivative or analogues thereof (e.g. YFP etc). Most preferred expression cassettes comprise a human GADD45α gene promoter operatively linked to a DNA sequence encoding a GFP or light emitting derivative thereof, and Intron 3 of the GADD45α gene.

In a first embodiment, the expression cassette according to the first aspect is preferably 5-31, (the cassette illustrated in Figure 9 as GD531). The nucleotide sequence of expression cassette 5-31 is shown in the sequence listing as SEQ ID No.2.

In a second embodiment, the expression cassette according to the first aspect is preferably 5-32 (the cassette illustrated in Figure 9 as GD532). The nucleotide sequence of expression cassette 5-32 is shown in the sequence listing as SEQ ID No.3.

In a third embodiment, the expression cassette according to the first aspect is preferably 5-33 (the cassette illustrated in Figure 9 as GD533) The nucleotide sequence of expression cassette 5-33 is shown in the sequence listing as SEQ ID No.4.

The recombinant vector according to the second aspect of the present invention may for example be a plasmid, cosmid or phage. Such recombinant vectors are of great utility when replicating the expression cassette. Furthermore, recombinant vectors are highly useful for transfecting cells with the expression cassette, and may also promote expression of the reporter protein.

Recombinant vectors may be designed such that the vector will autonomously replicate in the cytosol of the cell or can be used to integrate into the genome. In this case, elements that induce DNA replication may be required in the recombinant vector. Suitable elements are well known in the art, and for example, may be derived from pCEP4 (Invitrogen, 3 Fountain Drive, Inchinnan Business Park, Paisley, PA4 9RF, UK) pEGFP-N1 (BD Biosciences Clontech UK, 21 In Between Towns Road, Cowley, Oxford, OX4 LY, United Kingdom) or pCI and pSI (Promega UK ltd, Delta house, chilworth Science Park, Southampton SO16 7NS, UK).

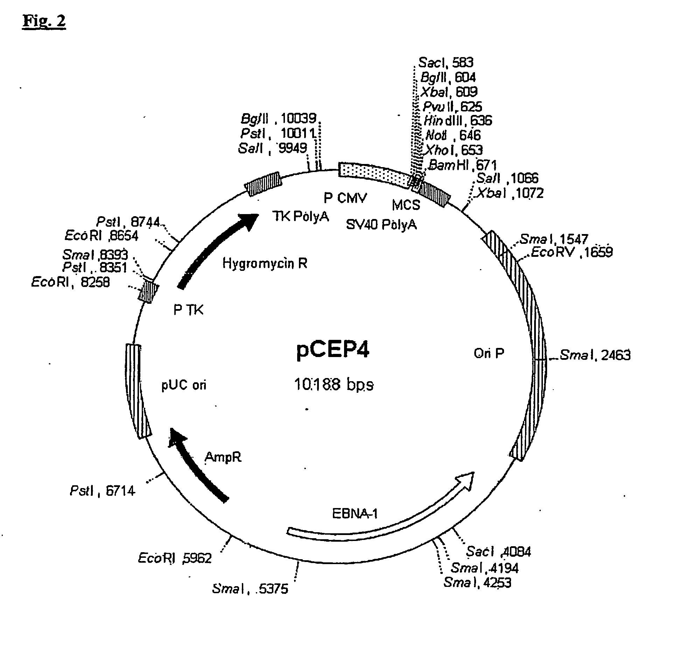

Such replicating vectors can give rise to multiple copies of the DNA molecule in a transformant and are therefore useful when over-expression (and thereby increased light emission) of the reporter protein is required. In addition, it is preferable that the vector is able to replicate in human, primate and/or canine cells. It is preferred that the vector comprises an origin of replication, and preferably, at least one selectable marker. The selectable marker may confer resistance to an antibiotic, for example, hygromycin or neomycin. Hence, a suitable element is derived from the pCEP4 plasmid (Invitrogen, 3 Fountain Drive, Inchinnan Business Park, Paisley, PA4 9RF, UK), which is illustrated in Figure 2.

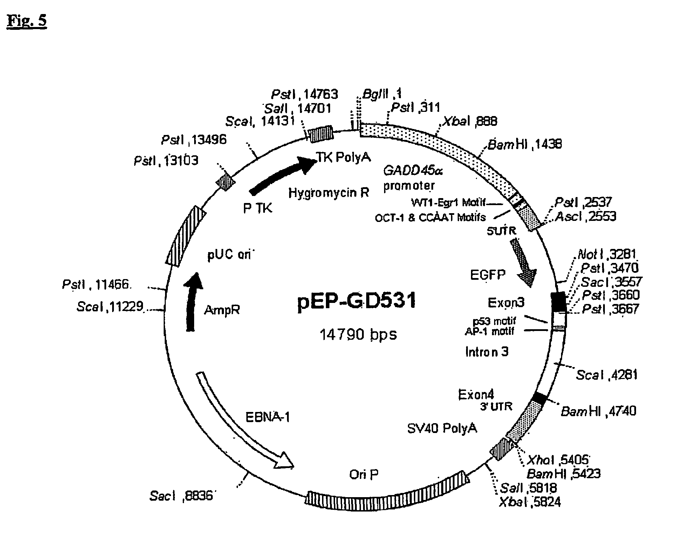

In a first embodiment, the recombinant vector according to the second aspect is preferably pEP-GD531, as illustrated in Figure 5.

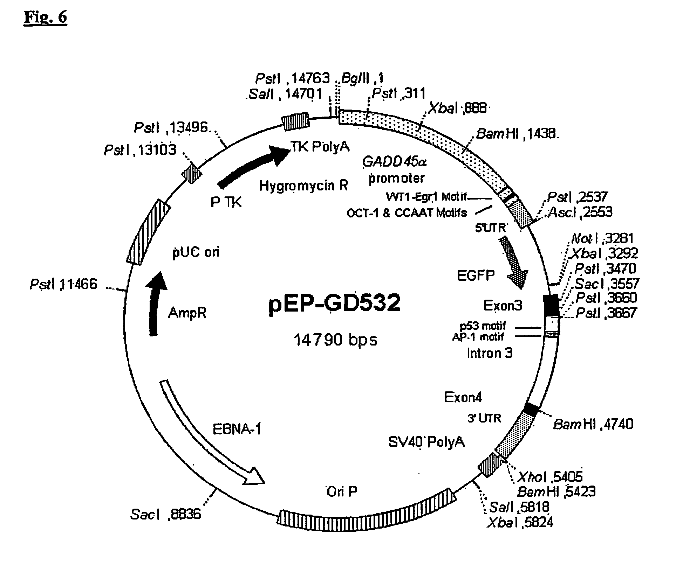

In a second embodiment, the recombinant vector according to the second aspect is preferably pEP-GD532, as illustrated in Figure 6.

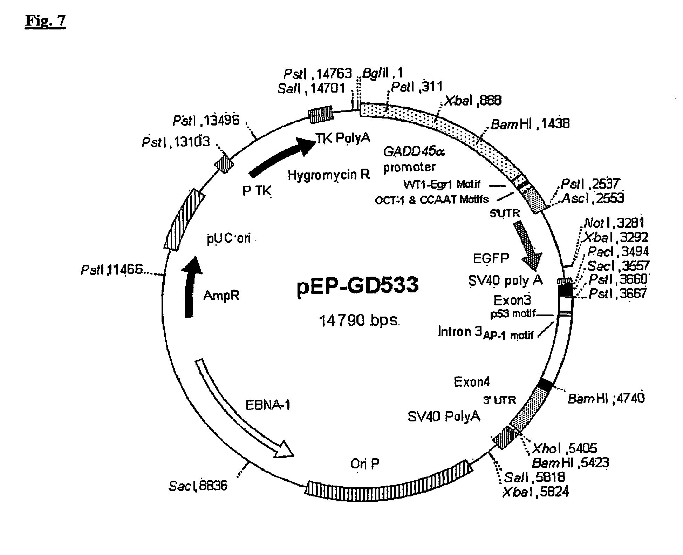

In a third embodiment, the recombinant vector according to the second aspect is preferably pEP-GD533, as illustrated in Figure 7.

According to the second aspect of the invention the recombinant vector is incorporated within a cell. It is preferred that the cell is eukaryotic. Such host cells may be mammalian derived cells and cell lines. Preferred mammalian cells include human, primate, murine or canine cells. The host cells may be lymphoma cells or cell lines, such as mouse lymphoma cells. The host cells may be immortalised, for example, lymphocytes.

Preferred host cells are human cell lines. Preferably, the host cells are human cell lines having a fully functional p53, for example, ML-1 (a human myeloid leukaemia cell line with wild-type p53), TK6 (a human lymphoblastoid cell line with wild-type p53). However, host cell lines of WI-L2-NS and WTK1 (both of which are sister lines of TK6 and have mutant p53 proteins) are also envisaged. These cell lines may all be found at the European Collection of Cell Cultures (ECACC General Office, CAMR, Porton Down, Salisbury, Wiltshire, SP4 OJG, United Kingdom).

The inventors have found that TK6 human cells are particularly preferred cell lines for use according to the method of the invention. While the inventors do not wish to be bound by any hypothesis, they believe that TK6 cells are most useful because they have a fully functional p53.

Host cells used for expression of the protein encoded by the DNA molecule are ideally stably transfected, although the use of unstably transfected (transient) cells is not precluded.

Transfected cells according to the third aspect of the invention may be formed by following procedures described in the Example. The cell is ideally a human cell line, for example TK6. Such transfected cells may be used according to the method of the fourth aspect of the invention to assess whether or not agents induce or potentiate DNA damage. GFP expression is induced in response to DNA damage and the light emitted by GFP may be easily measured using known appropriate techniques.

Most preferred cells according to the third aspect of the invention are TK6 cells transformed with the vector pEP-GAD532. These cells are referred to herein as GenTK-T01.

It is also envisaged that the expression cassette according to the invention may be integrated into the genome of a host cell. The skilled technician will appreciate suitable methods for integrating the cassette into the genome. For example, the expression cassette may be harboured on a retroviral vector, which in combination with a packaging cell line may produce helper-free recombinant retrovirus, which may then be introduced into the host cell. The cassette may then integrate itself into the genome. Examples of suitable helper-free retroviral vector systems include the pBabePuro plasmid with the BING retroviral packaging cell line [Kinsella and Nolan, 1996. Episomal Vectors Rapidly and Stably Produce High-Titer Recombinant Retroviruses. Human Gene Therapy. 7:1405-1413.]

The method of the fourth aspect of the invention is particularly useful for detecting agents that induce DNA damage at low concentrations. The methods may be used to screen compounds, such as candidate medicaments, food additives or cosmetics, to assess whether it is safe to expose a living organism, particularly people, to such compounds. Alternatively, the method of the fourth aspect of the invention may be employed to detect whether or not water supplies are contaminated by DNA damaging agents or agents that potentiate DNA damage. For instance, the methods may be used to monitor industrial effluents for the presence of pollutants that may lead to increased DNA damage in people or other organisms exposed to the pollution.

The method of the invention is preferably performed by growing cells transfected with a recombinant vector according to the second aspect of the invention (such as pEP-GD531, pEP-GD532, or pEP-GD533), incubating the cells with the agent which putatively causes DNA damage for a predetermined time and monitoring the expression of the light emitting reporter protein directly from a sample of the cells.

Cells are preferably grown in low fluorescence growth medium. This can obviate the need to wash the cells before measurements are made and therefore reduce the number of steps in the method further. For instance, preferred human cells according to the third aspect of the invention may be grown in a low fluorescent media. Suitable media may be a modified RPMI 1640 media and most preferably contains either low concentrations of, or preferably, no riboflavin or phenol red. The inventors have found that the use of such media is surprisingly useful in assays of fluorescent markers such as GFP. The use of such media reduces the "signal to noise ratio" when measuring fluorescence. Therefore, according to a fifth aspect of the invention there is provided a media that does not contain, or contains reduced levels (relative to standard cell culture media) of riboflavin or phenol red for use in fluorescent assays. Most preferably the media contains no riboflavin and no phenol red.

It is preferred that the media is modified such that it does not contain riboflavin. The use of such a modified media would be counter-intuitive to a skilled person because it would be understood that riboflavin is usually required in media. This is because most cells require riboflavin to be able to grow and survive. The inventors have realised that cells, which have been grown in conventional growth media containing fiboflavin, may be removed from such media; placed in media without riboflavin; and will remain viable for the purposes of conducting a reliable fluorescence assay. Cells left in conventional media (containing riboflavin and/or phenol red) tend to provide less satisfactory fluorescence values when tested according to the method of the fourth aspect of the invention (particularly when the reporter protein is GFP or a derivative thereof).

It is preferred that media is used according to the fifth aspect of the invention when conducting assays according to the fourth aspect of the invention. It is most preferred such media is used when performing a genotoxic test with GFP as the reporter.

A preferred media with low autofluorescence is a modified RPMI 1640 media that does not contain riboflavin or Phenol red. A most preferred media is disclosed in table 1 of Example 1.

Other media that could be modified for low autofluorescence include Dulbecco's Modified Eagle media (D-MEM), F-10 Nutrient mixture (Ham), F-12 Nutrient mixture (Ham), and Fischer's medium (GIBCO, Invitrogen, Paisley, UK). The most appropriate medium may be used for different cell types that could be used as a host for recombinant reporter vectors.
According to a preferred embodiment of the method of the invention TK6 cells may be transfected with pEP-GD531, pEP-GD532, or pEP-GD533, and grown in RPMI medium containing no riboflavin. A putative DNA damaging agent (e.g. a food additive or potential medicament or an agent contained within a water sample or effluent sample) may then be added to the medium containing the cells. The cells are then allowed to grow for a defined period of time after which a sample of the cells is removed and fluorescence measured therefrom.

Suitable methods of fluorescence detection and quantitation will be known to the skilled technician, and a method is described in the Examples. A preferred method of fluorescence detection is described in US 6, 509, 161. This method is particularly useful when the light emitting report is GFP or a derivative thereof.

According to a preferred embodiment of the method of the invention, fluorescence and absorbance readings may be recorded from TK6 cells transfected with pEP-GD531, pEP-GD532, or pEP-GD533, for example, from the well of a microplate. An example of a suitable microplate is a 96 well, black, clear-bottomed microplates, manufactured by Matrix ScreenMates, Cat. No. 4929, Apogent Discoveries, USA, or Corning (BV, Netherlands: Cat. No. 3651). Fluorescence and absorbance measurements may be recorded using a suitable microplate reader, for example, the Tecan Ultra-384 (Tecan UK Ltd.) microplate reader: excitation 485 nm / emission 535 nm with an additional dichroic mirror (reflectance 320 nm - 500 nm, transmission 520 nm - 800 nm). Absorbance may be measured through a suitable filter, for example, a 620 nm filter.

Most preferred protocols for conducting the method of the fourth aspect of the invention are described in Examples 1, 3 and 4.

From the fluorescence and absorbance measurements, "brightness values" and fluorescence induction ratios for cells according to the third aspect of the invention can be made as described in the examples.

There is background ("constitutive") expression of GFP from the GADD45α-EGFP constructs, thus the higher the cell density, the more fluorescent the culture. In order to correct for fluorescence increase that is consequent on growth, the fluorescence data (GFP) are divided by absorbance data (cell density) to give 'brightness units', i.e. the measure of average fluorescence per cell. This is independent of culture density. Accordingly, measurement of absorbance may be used primarily for normalisation of fluorescence signals rather than a measurement of the toxicity of the DNA damaging agent. Accordingly, it is envisaged that a secondary assay may be used in conjunction with the absorbance measurement in order to determine toxicity via cell viability and apoptosis. For example, using the Biovison Bioluminescence Cytotoxicity Assay (Biovison Incoperated, 2455-D Old Middlefield Way, Mountain View, California 94043, USA), or the Vybrant® Apoptosis Assay Kit (Molecular probes Inc., 29851 Willow Creek Road, Eugene, OR 97402, USA).

Occasionally, compounds show fluorescence themselves or induce cellular autofluorescence. Accordingly, when evaluating increased GFP expression the brightness values of a control cell line is subtracted from the brightness values of a reporter cell line. This removes the interference from the data. It is therefore preferred that methods according to the fourth aspect of the invention employ cells containing control vectors based upon reporter vectors according to the second aspect of the invention. Such control vectors may correspond to the reporter vector, but with a non-sense mutation introduced into the light emitting reporter gene.

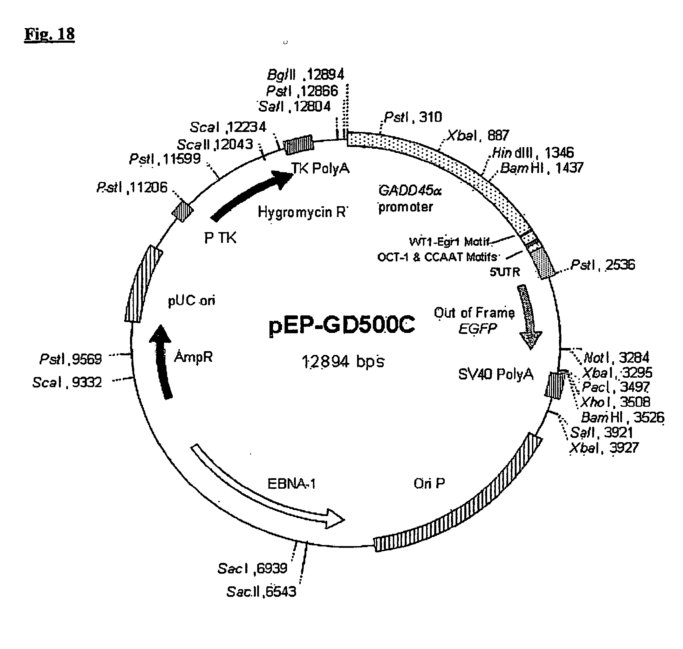

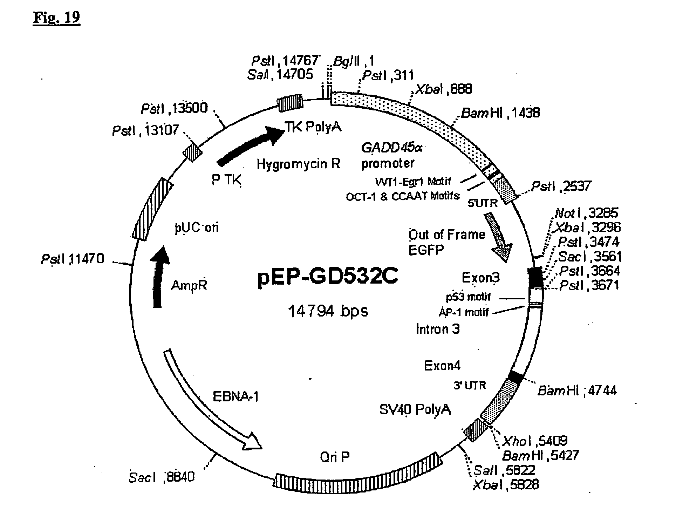

The recombinant control vector may preferably be the vectors described as pEP-GD500C or pEP-GD532C, as illustrated in Figures 18 and 19 respectively.

Preferred methods according to the fourth aspect of the invention will utilise cells according to the third aspect of the invention (e.g. GenTK-T01) amd a control cell line based upon TK6 cells transformed with the vector pEP-GD532C (referred to herein asGenTK-C01).

It will be appreciated that some non-genotoxic compounds can be chemically altered by cellular metabolism. In mammals this process is often called metabolic activation (MA). MA can convert certina non-genotoxic compounds (for example promutagens) into genotoxic compounds. Most frequently MA occurs in the liver. For this reason it is often preferred that genotoxicity tests are adapted such that assays of test compound are carried out in the presence and absence of liver extracts that are capable of metabolising a compound is if it were being metabolised *in vivo*. Example 4 illustrates a preferred method according to the fourth aspect of the invention which utilises a liver extract (known to the skilled person) called S9. Inclusion of such an extract allows assays to detect compounds that only become genotoxic after passage through the liver.

All of the features described herein (including any accompanying claims, abstract and drawings), and/or all of the steps of any method or process so disclosed, may be combined with any of the above aspects in any combination, except combinations where at least some of such features and/or steps are mutually exclusive.

Embodiments of the invention will now be further described, by way of example only, with reference to the following Examples and Figures in which:-
Figure 1 shows a restriction map of vector p*EGFP*-N1;
Figure 2 shows a restriction map of vector pCEP4;
Figure 3 shows a restriction map of vector pHG45-HC;
Figure 4 shows a restriction map of vector pEP-GD500;
Figure 5 shows a restriction map of vector pEP-GD531, a preferred recombinant vector according to the present invention;
Figure 6 shows a restriction map of vector pEP-GD532, a preferred recombinant vector according to the present invention;
Figure 7 shows a restriction map of vector pEP-GD533, a preferred recombinant vector according to the present invention;
Figure 8 shows a restriction map of vector pEP-GD534;
Figure 9 shows embodiments of reporter expression cassettes according to the invention;
Figure 10 shows the effect of MMS induction on *GADD45*α reporter cell lines;
Figure 11 shows the effect on brightness by MMS induction on *GADD45*α reporter cell lines;
Figure 12 shows methyl methanesulphonate induced fluorescence measured directly from a reporter cell line cultured in either RPMI 1640 with serum (A) or modified low autofluorescence medium with serum (B) as discussed in Example 2;
Figure 13 shows the growth of a human lymphoblastoid cell line in RPMI 1640 or modified low autofluorescence medium in Example 2;
Figure 14 shows the effect of MMS on relative cell density in a 96 well plate assay after 48 hours incubation in Example 3;
Figure 15 shows the effect of MMS on florescence induction ratios in a 96 well plate assay in Example 3;
Figure 16 shows the effect of Cyclophosphamide with and without S9 on the relative cell densitys of GenTK-C01 cells in Example 4;
Figure 17 shows the effect of Cyclophosphamide with and without S9 on the fluorescence induction in GenTK-C01 cells in Example 4;
Figure 18 shows a restriction map of a control vector named pEP-GD500C; and
Figure 19 shows a restriction map of a control vector named pEP-GD532C.

### Example 1

The following Example outlines the components that have been used in the construction of a series of *GADD45*α*-EGFP* Human cell culture DNA damage biosensors/reporters according to the first and second aspects of the invention. In addition, this Example describes the construction of the biosensors (cells according to the third aspect of the invention), and illustrates how they respond to a genotoxic agent, for example, methanesulphonic acid methyl ester (MMS) when utilised in a method according to the fourth aspect of the invention.

### 1.1 System Components:-

### (i) The Promoter - From GADD45α

The source of the *GADD45*α promotor is the plasmid pHG45-HC from Albert J. Fornace Jr., M.D. (National Cancer Institute, Building: 37, Room: 6144, National Institutes of Health, Bethesda, Maryland 20892, USA), which is illustrated in Figure 3.

### (ii) The Fluorescent Protein - Clontech's EGFP

The- source-of the Green fluorescent protein is the Clontech plasmid pEGFP-N1 (BD Biosciences Clontech UK, 21 In Between Towns Road, Cowley, Oxford, OX4 LY, United Kingdom), which is illustrated in Figure 1. pEGFP-N1 encodes a red-shifted variant of wild-type *GFP* that has been optimised for brighter fluorescence and higher expression in mammalian cells. (Excitation maximum = 488 nm; emission maximum = 507 nm.) The plasmid pEGFP-N1 encodes the GFPmut1 variant, which contains the double-amino-acid substitution of Phe-64 to Leu and Ser-65 to Thr. The coding sequence of the EGFP gene contains more than 190 silent base changes, which correspond to human codon-usage preferences.

### 1.2 Construction of Biosensor Reporter cassettes

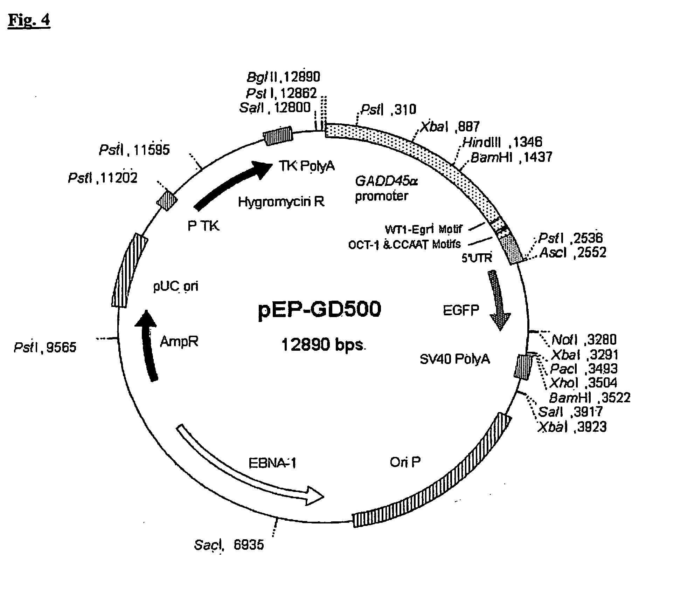

The construction of a set of recombinant vectors each containing a *GADD45*α-*EGFP* reporter cassette was carried out in several stages using the Invitrogen pCEP4 plasmid as a backbone, which is illustrated in Figure 2. Plasmid pCEP4 is an episomal mammalian expression vector that uses the cytomegalovirus (CMV) immediate early enhancer/promoter for high level transcription of recombinant genes inserted into the multiple cloning site. The Epstein-Barr Virus replication origin (oriP) and nuclear antigen (encoded by the EBNA-1 gene) is carried by this plasmid to permit extrachromosomal replication in human, primate and canine cells. pCEP4 also carries the hygromycin B resistance gene for stable selection in transfected cells.
Step 1: (Insertion of an *EGFP* module into pCEP4) - An *EGFP* module (consisting of the Human optimised Green Fluorescent Protein and the SV40 poly adenylation signal) was amplified by PCR from the Clontech plasmid p*EGFP*-N1 (BD Biosciences Clontech UK, 21 In Between Towns Road, Cowley, Oxford, OX4 LY, United Kingdom), which is shown in Figure 1. The *EGFP* gene is flanked by *Bgl* II-*Asc* I (5') and *Pac* I-*XhoI* (3') sites. This was inserted into *Bgl* II- *Xho* I cut pCEP4 (Invitrogen), which is shown in Figure 2. This step also resulted in the removal of the CMV promoter from pCEP4.
Step 2: (Insertion of *GADD45*α promoter) - The *GADD45*α 5' promoter sequence was PCR amplified (from a plasmid pHG45-HC, kindly donated by Albert J. Fornace Jr., M.D. (National Cancer Institute, Building: 37, Room: 6144, National Institutes of Health, Bethesda, Maryland 20892, USA), illustrated in Figure 3, from -2253bp up to and including the 5' UTR (untranslated region) and start codon (+298bp) with flanking *Bgl* II (5') and *AscI* I (3') sites. This was inserted into the plasmid resulting from step 1. The resultant plasmid was named pEP-GD500, which is shown in Figure 4.
Step 3: (Insertion of *GADD45*α gene sequence) - Various *GADD45*α gene sequences (3' end of gene) were amplified with cloning sites by PCR and cloned into the plasmid resulting from step 2 (pEP-GD500) to give four further reporter plasmids.

### These plasmids were named:-

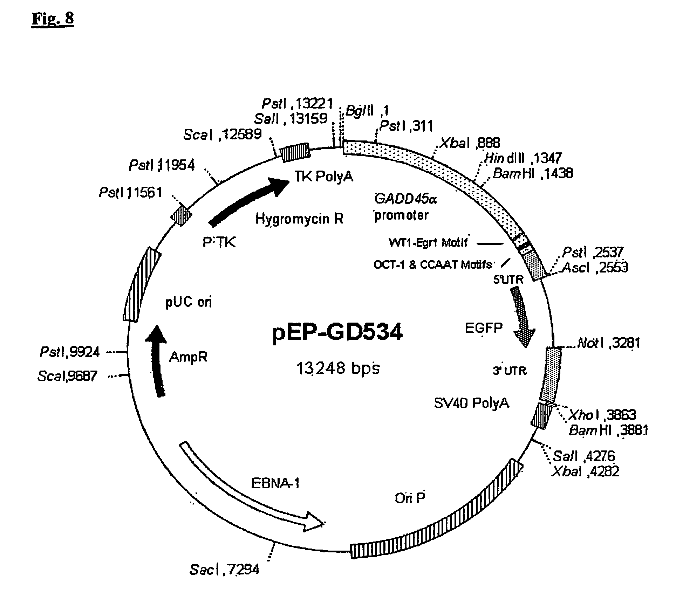

(i) pEP-GD531 (3' sequence from +1034 to +3149) - as shown in Figure 5. This plasmid includes all of Intron 3 of the Human GADD45 α gene, including the p53 binding motif;
(ii) pEP-GD532 (3' sequence from +1147 to +3149) - as shown in Figure 6. This plasmid includes all of Intron 3 of the Human GADD45 α gene, including the p53 binding motif;
(iii) pEP-GD533 (3' sequence from +1248 to +3149) as shown in Figure 7. This plasmid includes all of Intron 3 of the Human GADD45α gene, including the p53 binding motif; and
(iv) pEP-GD534 (3' sequence from +3155 to +3149) - as shown in Figure 8. This plasmid does not include all of Intron 3 of the Human GADD45α gene, and does not include the p53 binding motif. This plasmid includes the *GADD45*α 3' untranslated region.

### Cloning sites used for step 3:-

**pEP-GD531:** PCR product flanked by *Not* I (5') and *Xho* I (3'). Cloned into *Not* I - *Xho* I cut pEP-GD500.
**pEP-GD532:** PCR product flanked by *Eco*RV (5') *and Xho* I (3'). Cloned into *Hpa* I - *Xho* I cut pEP-GD500.
**pEP-GD533:** PCR product flanked by *Pac* I (5') and *Xho* I (3'). Cloned into *Pac* I - *Xho* I cut pEP-GD500.
**pEP-GD534:** PCR product flanked by *Not* I (5') and *Xho* I (3'). Cloned into *Not* I - *Xho* I cut pEP-GD500.
**Step 4:** (creation of control vectors) - Reporter plasmids were digested with *Asc* I and the 5' overhangs filled in with Klenow enzyme. The modified DNA fragments were then recirculised. This introduced a 4 bp frameshift in the EGFP gene. The resultant protein is non-functional. The resultant control plasmids are suffixed with the letter C and illustrated in Figures 18 and 19.

The DNA sequences of each of the expression cassettes in the plasmids pEP-GD500, pEP-GD531, pEP-GD532, pEP-GD533, and pEP-GD534 are shown in the sequence listing as SEQ ID No.1, SEQ ID No.2, SEQ ID No.3, SEQ ID No.4, and SEQ ID No.5, respectively.

Referring to Figure 9, there are shown the series of reporter cassettes in accordance with the invention each aligned underneath the Human GADD45α gene. The GADD45α *EGFP* reporter gene fusions of plasmids pEP-GD531, pEP-GD532, and pEP-GD533 were constructed in such a way that the overall length of the *GADD45*α *-EGFP* fusion is identical to the native *GADD45*α gene, as shown in Figure 9. In plasmids pEP-GD531 and pEP-GD532, the *GADD45*α DNA downstream of the hEGFP is transcribed into mRNA as well as the hEGFP itself, and is subject to splicing. In plasmid pEP-GD533, only the hEGFP gene is transcribed into mRNA. The transcription is stopped at the SV40 polyadenylation signal before the *GADD45*α gene sequence. These plasmids are in accordance with the first aspect of the invention. The pEP-GD534 cassette is much shorter and does not include the p53 motif in Intron 3 of the *GADD45*α gene.

### 1.3 Cell line selection and transfection

When considering a cell line as a host for a new mammalian DNA-damage reporter system, it was important to consider the ability of the chosen cell line to respond to genotoxic damage in a way that would allow the required response of the reporter. The p53 status and effects on downstream genes of two cells lines commonly used in molecular biology and/or genetic toxicology were explored and this work is described herein. These cell lines were:- (i) TK6, a human lymphoblastoid cell line (wt p53); and a sister line of TK6, (ii) WI-L2-NS (mutant p53 protein).

A dysfunctional p53 protein can lead to increased radioresistance (i.e. increased survival) and an increased sensitivity to mutagenic and clastogenic effects of radiation. While these properties make a cell line suitable for detecting mutation endpoints, the same properties imply that the induction of surrogate biomarkers for DNA damage in a cell line with a mutant p53 status would be at best poor. To this end, the TK6 cell line was chosen for the biosensor (i.e. a cell according to the third aspect of the invention). It is believed that additional data of scientific interest may be gained from the use of its sister cell lines with the recombinant *GADD45*α *-EGFP* reporter vector.

TK6 cells are transfected with the vectors by electroporation using a method adapted from Xia and Liber [Methods in Molecular biology, Vol.48: Animal Cell Electroporation and Electrofusion Protocols, 1995. Edited by J.A. Nickoloff. Humana Press Inc., Totowa, NJ, USA, Pages 151-160], and clones bearing the reporter plasmids are selected over two weeks with Hygromycin B.

TK6 cells transformed with pEP-GAD532 are referred to herin as GenTK-T01. An equivalent control cell line, termed GenTK-C01, was also produced in which the GFPreporter gene was deliberately placed out of frame. Accordingly GFP could not be expressed from this control cell line.

### 1.4 The Assay media - modified RPMI

Conventional media may be used in the methods of the invention. However the inventors have found that many traditional cell culture media are autofluorescent at the wavelengths associated with *EGFP* excitation (488nm max) and emission (520nm max). In this work, the major contributing factor of this fluorescence was found to be the vitamin riboflavin. It is therefore preferred that a modified low autofluorescent media is used.

A number of such media may be produced and, by way of example, the inventors developed a modified low autofluorescent RPMI 1640 media (see Table 1). This media represents a preferred media for use according to the fifth aspect of the invention that does not contain any riboflavin, and may be used with the biosensor, to facilitate direct fluorescence reading of biosensor cultures.

**Table 1: working concentrations of components of a low autofluorescent mammalian cell culture media.**

| Component | mg/L |
|---|---|
| **INORGANIC SALTS:** | |
| Ca(NO₃)₂ • 4H₂0 | 100.00 |
| KCl | 400.00 |
| MgSO₄ (anhyd.) | 48.84 |
| NaCl | 6000.00 |
| NaHCO₃ | 2000.00 |
| Na₂HPO₄ (anhyd.) | 800.00 |
| | |
| **OTHER COMPONENTS:** | |
| D-Glucose | 2000.00 |
| Glutathione (reduced) | 1.00 |
| | |
| **AMINO ACIDS:** | |
| L-Arginine HCl | 241.86 |
| L-Asparagine (free base) | 50.00 |
| L-Aspartic Acid | 20.00 |
| L-Cystine•2HCl | 65.20 |
| L-Glutamic Acid | 20.00 |
| Glycine | 10.00 |
| L-Histidine (free base) | 15.00 |
| L-Hydroxyproline | 20.00 |
| L-Isoleucine | 50.00 |
| L-Leucine | 50.00 |
| L-Lysine • HCl | 40.00 |
| L-Methionine | 15.00 |
| L-Phenylalanine | 15.00 |
| L-Proline | 20.00 |
| L-Serine | 30.00 |
| L-Threonine | 20.00 |
| L-Tryptophan | 5.00 |
| L-Tyrosine (disodium salt) | 28.83 |
| L-Valine | 20.00 |
| | |
| **VITAMINS**: | |
| D-Biotin | 0.20 |
| D-Ca Pantothenate | 0.25 |
| Choline Chloride | 3.00 |
| Folic Acid | 1.00 |
| i-Inositol | 35.00 |
| Nicotinamide | 1.00 |
| Para-aminobenzoic Acid | 1.00 |
| Pyridoxal HCl | 1.00 |
| Thiamine HCl | 1.00 |
| Vitamin B₁₂ | 0.005 |

### 1.5 The method of Analysis - Microplate reader

Fluorescence and absorbance data was collected from microplates using the Tecan Ultra-384 (Tecan UK Ltd.) microplate reader: excitation 485 nm / emission 535 nm with an additional dichroic mirror (reflectance 320 nm - 500 nm, transmission 520 nm - 800 nm). Absorbance was measured through a 620 nm filter. The data were transported into a Microsoft Excel spreadsheet, and converted to graphical data. The absorbance data give an indication of reduction in proliferative potential and these data were normalised to the untreated control (=100% growth). Fluorescence data were divided by absorbance data to give 'brightness units', the measure of average GFP induction per cell. These data were normalised to the untreated control (=1). In this way, one can distinguish between a small number of highly fluorescent cells and a large number of weakly fluorescent cells. In order to correct for induced cellular autofluorescence and intrinsic compound fluorescence, the brightness values for the untransfected TK6 cell line were subtracted from those of the five cell lines bearing a reporter plasmid. This makes visual assessment of the data more reliable. All the data were checked with and without this correction. From this "brightness" data the fluorescence induction ratios were calculated.

### 1.6 MMS Induction of the Biosensor

As an example of reporter induction, six cell lines (TK6, without a plasmid, and bearing one of the five reporter plasmids) were treated with varying concentrations of MMS. MMS is known to those skilled in he art as a genotoxic and cytotoxic agent and may be used as a positive control when using the method of the invention.

1×10⁶ cells in 1 ml of modified RPMI 1640 media (see 1.4 above)+ 10% horse Serum were incubated overnight (37°C, 5% CO₂, 100% humidity) with 0µg/ml, 6.25µg/ml, 12.5µg/ml, and 25µg/ml MMS.

After incubation, the cells were washed in PBS, resuspended in 300µl PBS, and then split into two wells of a 96 well, black, clear-bottomed microplates. For example Matrix ScreenMates, Cat. No. 4929, Apogent Discoveries, USA, or Corning (BV, Netherlands: Cat. No.3651). Fluorescence and absorbance readings were recorded and fluorescence induction ratios were calculated.

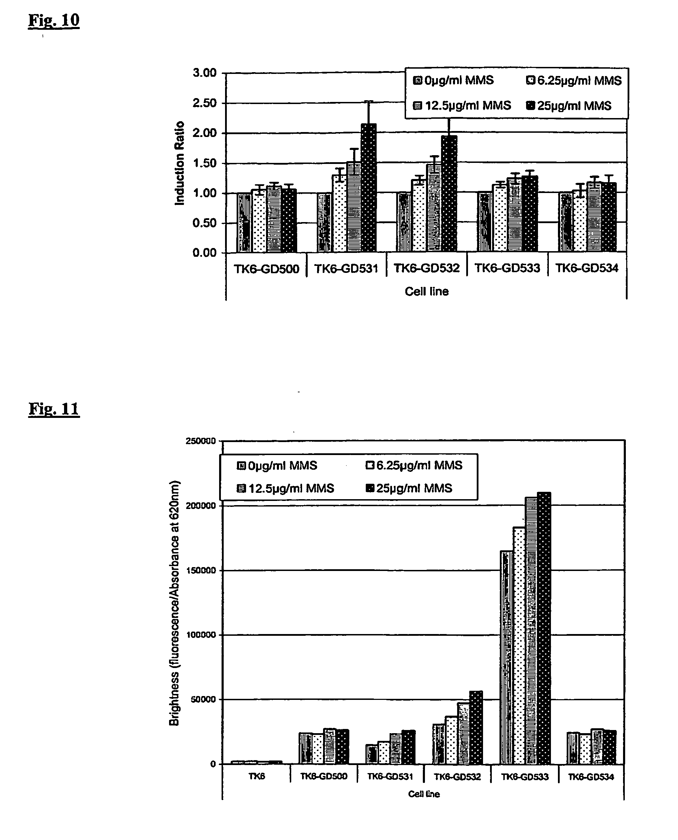

This was repeated three times and the average inductions and standard errors were calculated. The results are illustrated in Figure 10.

Referring to Figure 11, there is shown unprocessed brightness values from the five reporter cell lines and the parent untransfected cell line TK6. Brightness values were calculated for each cell line-MMS combination by measuring the total GFP fluorescence and then normalising this with absorbance at 620nm. Hence, it will be appreciated that each of the plasmids pEP-GD531, pEP-GD532, and pEP-GD533 show an increase in background fluorescence as compared to the untransfected parent cell line TK6, and these cell lines showed a substantial degree of dose dependent increase in fluorescence in the human cell line TK6 when treated with MMS. In contrast while the cell lines bearing the plasmid pEP-GD500 or pEP-GD534 show an increase in background fluorescence as compared to TK6, this fluorescence is not further induced by treatment with MMS. While the inventors do not wish to be bound by any hypothesis, they believe that the addition of the GADD45α 3' untranslated region in plasmid pEP-GD534 stabilises the resultant mRNA, leading to a higher background level of fluorescence in cell lines bearing this plasmid as compared to cell lines bearing the plasmid pEP-GD500.

In conclusion, the present invention uses the regulatory sequences of the human DNA-Damage inducible gene *GADD45*α to control the production of *EGFP* in the p53 wild-type human lymphoblastiod cell line TK6. The human gene *GADD45*α stimulates DNA excision repair *in vitro* and inhibits entry of cells into S phase. The reporters in accordance with the invention not only use the upstream promoter of the *GADD45*α gene, but also incorporate the gene regulatory elements found within the genomic gene sequence itself. This includes a putative p53 binding site in the third Intron of the *GADD45*α gene. Surprisingly, the use of these sequences enhances the response of the biosensors to genotoxic stress. This reporter system can be analysed for fluorescence induction after only 24 hours by simply measuring the fluorescence of the test culture. The cells may be subjected to an agent, and the expression of the light emitting reporter protein (GFP) from the cell indicates that the agents cause DNA damage. It is believed that this biosensor has applications in short term pre-regulatory drug screening.

### Example 2

Further experiments were conducted to evaluate the effects of autofluorescence caused by conventional media in order that preferred media may be developed for use in the method according to the fourth aspect of the invention.

The constructs, cells and protocols described in Example 1 were used unless indicated to the contrary.

As-well-as riboflavin (see 1.4) the inventors established that a second major contribution factor to cell culture media autofluorescence was phenol red (a pH indicator dye commonly used in culture media). The fluorescence of phenol red also varies with pH. Therefore it is preferred that media used according to the invention is formulated as a modified low autofluorescence medium whereby phenol red or riboflavin, but preferably both phenol red and riboflavin, is omitted (see table 1).

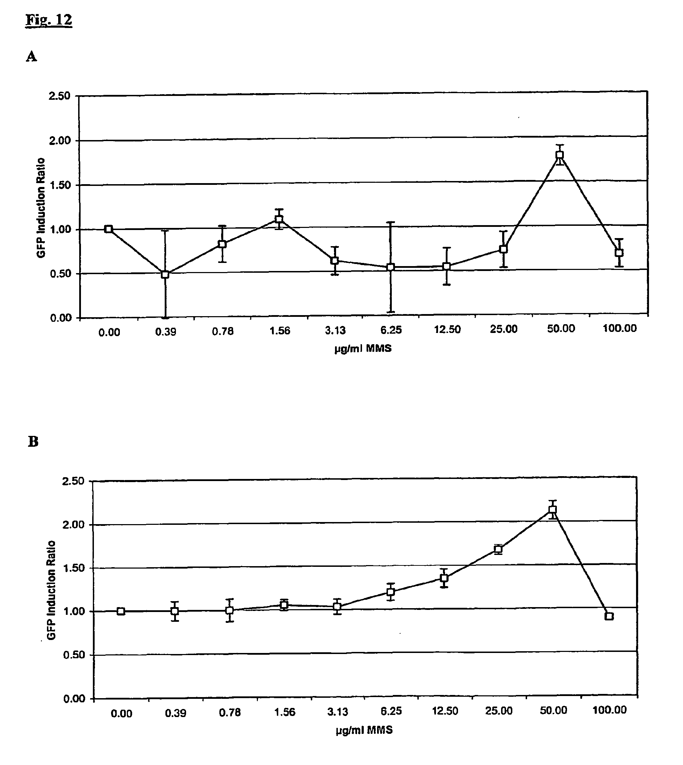

Referring to figure 12, the use of conventional RPMI 1640 medium decreased fluorescence signal to noise ratios, and increased variability of fluorescence measurements. Accordingly the use of normal RPMI 1640 media was not optimal for direct fluorescence readings. In contrast, the use of a modified low autofluorescent cell culture media (corresponding to normal media save riboflavain and phenol red was omitted) facilitated the direct measurement of genotoxin induced fluorescence readings.

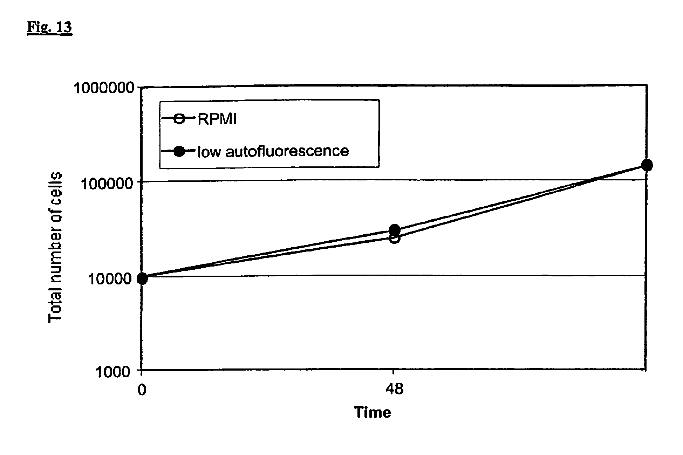

Referring to figure 13, the omission of both riboflavin and phenol red from media was found to result in a low autofluorescence medium that did not affect cell growth over 96 hours, and four cell divisions. Accordingly such a media is a preferred media for use according to the fifth aspect of the invention.

It will therefore be appreciated that it is preferred that media used in the method according to the fourth aspect of the invention may comprise conventional media (e.g. RPMI 1640) but is preferably modified, according to the fifth aspect of the invention, such that it does not contain Phenol Red or riboflavin.

### Example 3

Further development work was conducted to develop a most preferred protocol for conducting the method according to the fourth aspect of the invention in 96 well plates.

The constructs, cells and protocols described in Example 1 were used unless indicated to the contrary.

A preferred method of testing for DNA damage according to the fourth aspect of the invention comprises the steps of : (1) preparing a microplate for use in an assay; (2) conducting the assay in the microplates; (3) collecting and analysing the data; and (4) making a judgment on DNA damage and the consequences.

Details of steps (1) - (4) for conducting a most preferred test for DNA damage are given below.

### 3.1 Microplate preparation.

Assays were carried out in 96 well, black, clear-bottomed microplates. For example Matrix ScreenMates, Cat. No. 4929, Apogent Discoveries, USA, or Corning (BV, Netherlands: Cat. No. 3651). A number of alternative microplates were assessed, though the variable background absorbance and fluorescence both within and between plates from individual manufacturers were found to be variable and in several cases unacceptable. It will therefore be appreciated that microplates used according to the invention preferably have consistent absorbance and fluorescence between plates and batches thereof. It will be appreciated that a skilled person should give careful consideration when selecting a suitable microplate.

The assay plates can be filled using a liquid handling robot. For example the MicroLabS single probe, from Hamilton GB Ltd., Birmingham or a Genesis 8-probe robot (Tecan UK Ltd. Theale. UK). Microplates can also be filled rapidly and effectively using a multi-channel pipette.

### 3.2 Assay

The following standard protocol may be followed. A 2 mM or 1000µg/ml (whichever is lowest) stock of a test chemical, or sample containing an agent that putatively caused DNA damage, was prepared in 2% v/v aqueous DMSO, and used to make 2 identical dilution series across a 96 well microplate and a 'control' (see below). To achieve this, 150 microlitres of the test chemical solution were put into 2 microplate wells. Each sample was serially diluted by transferring 75 microlitres into 75 microlitres of 2% DMSO, mixing, and then taking 75 microlitres out and into the next well. This produced 9 serial dilutions of 75 microlitres each. The final top concentration of test chemical/sample is 1 mM or 500 µg/ml on the microplate.

Controls were added as follows:
a. Test compound/ sample containing agent in assay medium alone, to provide information on compound absorbance/fluorescence
b. Human cell cultures diluted with 1% DMSO alone, to give a measure of maximum proliferative potential
c. MMS as a genotoxicity and cytotoxicity control: 'high' = 50 µg/ml, 'low' = 10 µg/ml v/v
d. 2% DMSO diluent alone, to confirm lack of diluent absorbance/fluorescence
e. Growth medium alone, to confirm sterility/lack of contamination

Exponentially growing cultures of cells lines according to the invention (e.g. GenTK-C01 and GenTK-T01) were washed in D-PBS and suspended at a density of 2 x 10⁶ cells/ml in double strength low autofluorescent assay medium according to the invention. 75 microlitres of the cell suspension were added to each well of the diluted chemical: GenTK-C01 to one series and GenTK-T01 to the second, and to appropriate standards and controls. After the plates were filled, they were sealed using either a gas permeable membrane (for example Breath-easy, Diversified Biotech, USA) or a plastic lid, and then incubated without shaking, for 24 hours at 37°C, 5% CO₂, 95% humidity.

### 3.3 Data collection and handling.

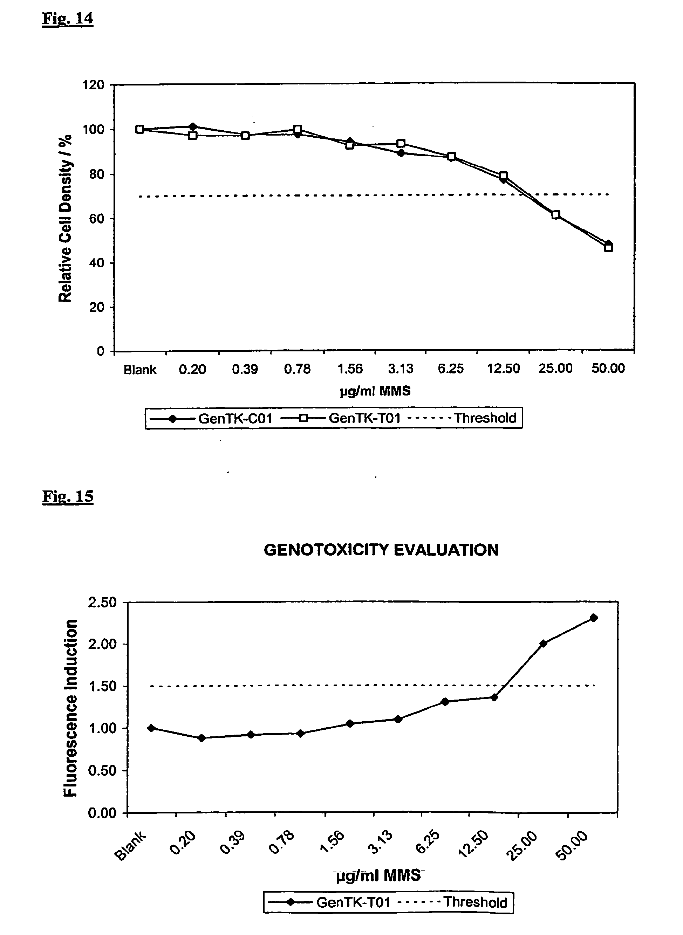

Following 24 hour incubation, fluorescence and absorbance data were collected from the microplates. Two different microplate readers which combine fluorescence and absorbance functionality have been used, and comparable data were obtained. These were a Tecan Ultra-384 (Tecan UK Ltd.): excitation 485 nm / emission 535 nm with an additional dichroic mirror (reflectance 320 nm - 500 nm, transmission 520 nm - 800 nm), and a Wallac Victor2 (PerkinElmer Life and Analytical Sciences, Monza, Italy): excitation 485 nm / emission 535 nm. Absorbance was measured through a 620 nm filter in both instruments. Microplates were re-sealed using either a gas permeable membrane (for example Breath-easy, Diversified Biotech, USA) or a plastic lid, and then incubated without shaking, for a further 24 hours at 37°C, 5% CO₂, 95% humidity. Further absorbance and fluorescence measurements were then collected. The data were transported into a Microsoft Excel spreadsheet, and converted to graphical data (see typical data in Figs 14 and 15 for Example 2). Data processing is minimal: absorbance data give an indication of reduction in proliferative potential and these data were normalised to the untreated control (=100% growth). Fluorescence data were divided by absorbance data to give 'brightness units', the measure of average GFP induction per cell. These data were normalised to the untreated control (=1). In this way, one can distinguish between a small number of highly fluorescent cells and a large number of weakly fluorescent cells. In order to correct for induced cellular autofluorescence and intrinsic compound fluorescence, the brightness values for the GenTK-C01 cell line were subtracted from those of GenTK-T01. This makes visual assessment of the data more reliable. All the data were checked with and without this correction, and the decision (see below), on whether or not a compound was classified as being genotoxic, was not affected.

### 3.4 Decision thresholds.

It is useful to have clear definitions of positive and negative results from routine assays and such definitions have been derived, taking into account the maximum noise in the system and data from chemicals where there is a clear consensus on genotoxicity and mechanism of action. Naturally it is also possible for users to inspect the numerical and graphical data and draw their own conclusions. For example an upward trend in genotoxicity data that did not cross the threshold might still distinguish two compounds. The decision thresholds were set as follows:

The cytotoxicity threshold is set at 80 % of the cell density reached by the untreated control cells. This is greater than 3 times the standard deviation of the background. A positive cytotoxicity result (+) is concluded if 1 or 2 compound dilutions produce a final cell density lower than the 80% threshold. A strong positive cytotoxicity result positive (++) is concluded when either (i) three or more compound dilutions produce a final cell density lower than the 80% threshold or (ii) at least one compound dilution produces a final cell density lower than a 60% threshold. A negative result (-) is concluded when no compound dilutions produce a final cell density lower than the 80% threshold. The lowest effective concentration (LEC) is the lowest test compound concentration that produces a final cell density below the 80% threshold.

The compound absorbance control allows a warning to be generated if a test compound is significantly absorbing. If the ratio of the absorbance of the compound control well to a well filled with media alone is > 2, there is a risk of interference with interpretation. The cytotoxicity controls indicate that the cell lines are behaving normally. The 'high' MMS standard should reduce the final cell density to below the 80% threshold, and should be a lower value than the 'low' standard.

The genotoxic threshold is set at a relative GFP induction of 1.5 (i.e. a 50% increase). This is greater than 3 times the standard deviation of the background. A positive genotoxicity result (+) is concluded if 1 or 2 compound dilutions produce a relative GFP induction greater than the 1.5 threshold. A strong positive genotoxicity result (++) is concluded if either (i) three or more compound dilutions produce a relative GFP induction greater than the 1.5 threshold or (ii) at least one compound dilution produces a relative GFP induction greater than a 1.8 threshold. A negative genotoxicity result (-) is concluded where no compound dilutions produce a relative GFP induction greater than the 1.5 threshold. The LEC is the lowest test compound concentration that produces a relative GFP induction greater than the 1.5 threshold. The genotoxic controls demonstrate that the cell lines are responding normally to DNA damage. The 'high' MMS standard must produce a fluorescence induction > 2, and be a greater value than the 'low' MMS standard. Anomalous brightness data is generated when the toxicity leads to a final cell density less than 30% that of the blank. Genotoxicity data is not calculated above this toxicity threshold. Compounds that tested negative for genotoxicity, were re-tested up to 10mM or 5000 µg/ml, or to the limit of solubility or cytotoxicity.

The compound fluorescence control allows a warning to be generated when a compound is highly auto-fluorescent. If the ratio of the fluorescence of the compound control well to a media filled well is >5, there is a risk of interference with interpretation. In these cases, fluorescence polarisation can be used to distinguish GFP from other sources of fluorescence (Knight *et al.*, 2000, 2002). The Tecan instrument has this facility. Occasionally, compounds though not fluorescent themselves, induce cellular autofluorescence. This is apparent from rising brightness in the control (GenTK-C01) cell line in the absence of fluorescence from the chemical-only control. The routine subtraction of GenTK-C01 from GenTK-T01 data removes this interference from the data.

### Example 4

Further development work was conducted to develop another most preferred protocol for conducting the method according to the fourth aspect of the invention in 24 well plates with and without S9 metabolic activation.

S9 is a liver extract (known to the skilled person) that makes it possible to discriminate between genotoxic compounds and some non-genotoxic compounds that may be chemically altered by hepatocyte metabolism to generate a genotoxic compound *in vivo*.

The constructs, cells and protocols described in Example 1 were used unless indicated to the contrary.

A preferred method of testing for DNA damage according to the fourth aspect of the invention comprises the steps of : (1) preparing a 24 well plate for use in an assay with metabolic activation; (2) conducting the assay in 24 well plates and processing samples in to 96 well plates; (3) collecting and analysing the data; and (4) making a judgment on DNA damage and the consequences.

Details of steps (1) - (4) for conducting a most preferred test for DNA damage are given below.

### 4.1 Microplate preparation.

Assays were carried out in 24 well plates (Corning BV, Schiphol-Rijk The Netherlands) and 96 well, black, clear-bottomed microplates. For example Matrix ScreenMates, Cat. No. 4929, Apogent Discoveries, USA, or Corning (BV, Netherlands: Cat. No. 3651). A number of alternative microplates were assessed, though the variable background absorbance and fluorescence both within and between plates from individual manufacturers were generally unacceptable, leading to the currently preferred choice. It will therefore be appreciated that microplates used according to the invention preferably have consistent absorbance and fluorescence between plates and batches thereof.

The assay plates can be filled using a liquid handling robot. For example the MicroLabS single probe, from Hamilton GB Ltd., Birmingham or a Genesis 8-probe robot (Tecan UK Ltd. Theale. UK). Microplates can also be filled rapidly and effectively using a multi-channel pipette.

### 4.2 Assay

The following standard protocol may be followed. To each row of a 24 well plate 1ml of the following Cell line media combinations were added to each well:
i) 1 ×10⁶ cells/ml GenTK-C01 in RPMI 1640 media
ii) 1 ×10⁶ cells/ml GenTK-T01 in RPMI 1640 media
iii) 1 × 10⁶ cells/ml GenTK-C01 in RPMI 1640 media + 10% S9 mix
iv) 1 × 10⁶ cells/ml GenTK-T01 in RPMI 1640 media +10% S9 mix

The RPMI 1640 media is preferably a modified media as defined by the fifth aspect of the invention (e.g. the media disclosed in Table 1).

A 100mg/ml stock of a test chemical, or sample containing an agent that putatively caused DNA damage, was prepared in 100% v/v aqueous DMSO, and used to make 4 identical dilution series across a 24 well microplate and a 'control' (see below). To achieve this, 200 microlitres of the test chemical solution were put into a sterile 7ml glass vial or 1.5 ml microfuge tube. Each sample was serially diluted by transferring 100 microlitres into 100 microlitres of 100% DMSO, mixing, and then taking 100 microlitres out and into the next well. This produced 3 serial dilutions of 100 microlitres each. To each well of a 24 well plate The final top concentration of test chemical/sample is 1 mg/ml on the 24 well plate.

Controls were added as follows:
a. Human cell cultures diluted with 10µl 100% DMSO alone, to give a measure of maximum proliferative potential
b. Cyclophosphamide as a positive genotoxicity and cytotoxicity control at 30 µg/ml in the presence of S9.

After the plates were filled, they were sealed using either a gas permeable membrane (for example Breath-easy, Diversified Biotech, USA) or a plastic lid, and then incubated without shaking, for 24 hours at 37°C, 5% CO₂, 95% humidity. Following 24 hours incubation cells were transferred to 1.5 ml microfuge tubes and collected by centrifugation at 2500 rcf for 5 minutes. Cells were washed in 500 µl pre-warmed D-PBS (Sigma-Aldrich, Gillingham, UK) and collected by centrifugation as before. Cells were resuspended in 150 µl D-PBS, and cell suspensions transferred to a well of an optically clear bottom, black polystyrene 96 well microplate (Matrix Technologies, Wilmslow, UK). As blanks, 150 µl of D-PBS were added to 2 wells.

### 4.3 Data collection and handling.

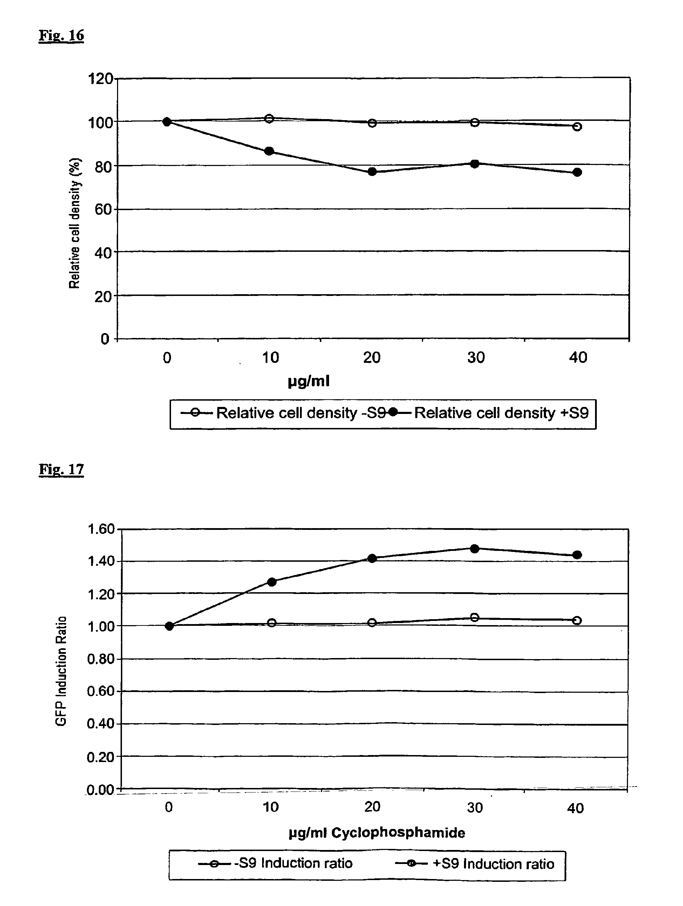

After transferring cell suspensions to 96 well plates fluorescence and absorbance data were collected. Two different microplate readers which combine fluorescence and absorbance functionality have been used, and comparable data were obtained. These were a Tecan Ultra-384 (Tecan UK Ltd.): excitation 485 nm / emission 535 nm with an additional dichroic mirror (reflectance 320 nm - 500 nm, transmission 520 nm - 800 nm), and a Wallac Victor2 (PerkinElmer Life and Analytical Sciences, Monza, Italy): excitation 485 nm / emission 535 nm. Absorbance was measured through a 620 nm filter in both instruments. The data were transported into a Microsoft Excel spreadsheet, and converted to graphical data (see typical data in Figs 16 and 17 for Example 3). Data processing is minimal: absorbance data give an indication of reduction in proliferative potential and these data were normalised to the untreated control (=100% growth). Fluorescence data were divided by absorbance data to give 'brightness units', the measure of average GFP induction per cell. These data were normalised to the untreated control (=1). In this way, one can distinguish between a small number of highly fluorescent cells and a large number of weakly fluorescent cells. In order to correct for induced cellular autofluorescence and intrinsic compound fluorescence, the brightness values for the GenTK-C01 cell line were subtracted from those of GenTK-T01. This makes visual assessment of the data more reliable. All the data were checked with and without this correction, and the decision (see below), on whether or not a compound was classified as being genotoxic, was not affected.

### 4.4 Decision thresholds.

It is useful to have clear definitions of positive and negative results from routine assays and such definitions have been derived, taking into account the maximum noise in the system and data from chemicals where there is a clear consensus on genotoxicity and mechanism of action. Naturally it is also possible for users to inspect the numerical and graphical data and draw their own conclusions. For example an upward trend in genotoxicity data that did not cross the threshold might still distinguish two compounds. The decision thresholds were set as follows:

The cytotoxicity threshold is set at 80 % of the cell density reached by the untreated control cells. This is greater than 3 times the standard deviation of the background. A positive cytotoxicity result (+) is concluded if 1 or 2 compound dilutions produce a final cell density lower than the 80% threshold. A strong positive cytotoxicity result positive (++) is concluded when either (i) three or more compound dilutions produce a final cell density lower than the 80% threshold or (ii) at least one compound dilution produces a final cell density lower than a 60% threshold. A negative result (-) is concluded when no compound dilutions produce a final cell density lower than the 80% threshold. The lowest effective concentration (LEC) is the lowest test compound concentration that produces a final cell density below the 80% threshold.

The compound absorbance control allows a warning to be generated if a test compound is significantly absorbing. If the ratio of the absorbance of the compound control well to a well filled with media alone is > 2, there is a risk of interference with interpretation. The cytotoxicity controls indicate that the cell lines are behaving normally.

The genotoxic threshold is set at a relative GFP induction of 1.3 (i.e. a 30% increase). This is greater than 3 times the standard deviation of the background. A positive genotoxicity result (+) is concluded if 1 or 2 compound dilutions produce a relative GFP induction greater than the 1.3 threshold. A strong positive genotoxicity result (++) is concluded if either (i) three or more compound dilutions produce a relative GFP induction greater than the 1.3 threshold or (ii) at least one compound dilution produces a relative GFP induction greater than a 1.6 threshold. A negative genotoxicity result (-) is concluded where no compound dilutions produce a relative GFP induction greater than the 1.3 threshold. The LEC is the lowest test compound concentration that produces a relative GFP induction greater than the 1.3 threshold. The genotoxic controls demonstrate that the cell lines are responding normally to DNA damage. The Cyclophosphomide standard must produce a fluorescence induction > 1.3 with S9, and not produce a fluorescence induction > 1.1 without S9. Anomalous brightness data is generated when the toxicity leads to a final cell density less than 30% that of the blank. Genotoxicity data is not calculated above this toxicity threshold. Compounds that tested negative for genotoxicity, were re-tested up to 10mM or 5000 µg/ml, or to the limit of solubility or cytotoxicity.

## Claims

1. An expression cassette comprising a DNA sequence encoding a reporter protein, which DNA sequence is operatively linked to a human GADD45α gene promoter and an additional human GADD45α gene regulatory element, the DNA sequence being place 3' to the GADD45α gene promoter **characterised in that** the regulatory element comprises a region of Intron 3 of the GADD45α gene including putative p53 binding motif.

2. An expression cassette according to claim 1, wherein the regulatory element comprises Exon 1, Exon 2, Exon 3, and/or Exon 4 of the GADD45α gene, or a region thereof, or any combination thereof.

3. An expression cassette according to claim 2, wherein the regulatory element comprises a region of Exon 1 of the GADD45α gene, a region of Exon 3 of the GADD45α gene, and a region of Exon 4 of the GADD45α gene.

4. An expression cassette according to any preceding claim, wherein the regulatory element additionally comprises Intron and/or Intron 2 of the GADD45α gene, or a region thereof.

5. An expression cassette according to any preceding claim, wherein the regulatory element comprises Intron 3 of the GADD45α gene.

6. An expression cassette according to claim 5 wherein the regulatory element comprises a putative AP-1 motif.

7. An expression cassette according to any preceding claim, wherein the DNA sequence encodes a light emitting reporter protein.

8. An expression cassette according to any preceding claim, wherein the DNA sequence encodes Green Fluorescent Protein (GFP), and light emitting derivatives thereof.

9. An expression cassette according to any preceding claim, wherein the DNA sequence encodes a human enhanced GFP (hEGFP), which consists of the GFP wild type containing a double amino acid substitution of Phe-64 to Leu, and SER-65 to Thr.

10. An expression cassette GD531, as illustrated in Figure 9, and as provided in SEQ ID No.2.

11. An expression cassette GD532, as illustrated in Figure 9, and as provided in SEQ ID No.3.

12. An expression cassette GD533, as illustrated in Figure 9, and as provided in SEQ ID No.4.

13. A recombinant vector comprising an expression cassette according to any one of claims 1-12.

14. A recombinant vector according to claim 13, wherein the vector comprises DNA from the pCEP4 plasmid.

15. A recombinant vector PEP-GD531, as illustrated in Figure 5.

16. A recombinant vector PEP-GD532, as illustrated in Figure 6.

17. A recombinant vector PEP-GD422, as illustrated in Figure 7.

18. A cell containing a recombinant vector according to any one of claims 14 to 17.

19. A cell according to claim 18, wherein the cell is a human cell.

20. A cell according to claim 19, wherein the cell is a human cell having a fully functional p53.

21. A cell according to claim 20, wherein the cell is a TK6 human cell line.

22. An in vitro method of detecting for the presence of an agent that causes or potentiates DNA damage comprising subjecting a cell according to any one of claims 18 to 21 to an agent; and monitoring the expression of the reporter protein from the cell.

23. The method according to claim 22, wherein the agent is further screened to assess whether it is safe to expose a living organism to the agent.

24. The method according to either claim 22 or claim 23, wherein the agent is a candidate medicament, food additive or cosmetic.

25. The method according to any one of claims 22 to 24, wherein the expression of a light emitting reporter protein from the cell is monitored.

26. The method according to claim 26, wherein the light emitting reporter protein is Green Fluorescent Protein,

27. The method according to claim 26, comprising growing cells transfected with a recombinant vector according to any one of claims 14-18, incubating the cells with the agent for a pre-determined time, and monitoring the expression of the Green Fluorescent Protein directly from a sample of the cells.

28. The method according to any one of claims 22 to 27, wherein the cells are grown in a low fluorescence growth medium.

29. The method according to claim 28, wherein the low fluorescence growth medium is an RPMI medium containing no riboflavin.

30. The method according to claim 28 or 29, wherein the low fluorescence growth medium is an RPMI medium containing no phenol red.

31. The method according to any one of claims 25 to 30, wherein fluorescence is determined by comparing fluorescence from a cell according to claims 18 to 21 with fluorescence from a control cell comprising a vector in which the nucleic acid encoding the light emitting reporter is placed out of frame.

## Patentansprüche

1. Expressionskassette, umfassend eine DNA-Sequenz, die ein Reporterprotein kodiert, wobei die DNA-Sequenz wirksam mit einem menschlichen GADD45α-Genpromotor und einem zusätzlichen GADD45α-Gen-regulatorischen Element verknüpft ist, wobei die DNA-Sequenz 3 zu dem GADD45α-Genpromotor plaziert ist, **dadurch gekennzeichnet, daß** das regulatorische Element eine Region des Introns 3 des GADD45α-Gens, einschließlich eines mutmaßlichen p53-Bindungsmotivs, umfaßt.

2. Expressionskassette nach Anspruch 1, worin das regulatorische Element Exon 1, Exon 2, Exon 3 und/oder Exon 4 des GADD45α-Gens oder eine Region davon umfaßt, oder irgendeine Kombination daraus.

3. Expressionskassette nach Anspruch 2, worin das regulatorische Element eine Region des Exons 1 des GADD45α-Gens, eine Region des Exons 3 des GADD45α-Gens und eine Region des Exons 4 des GADD45α-Gens umfaßt.

4. Expressionskassette nach einem vorangehenden Anspruch, worin das regulatorische Element zusätzlich Intron 1 und/oder Intron 2 des GADD45α-Gens oder eine Region davon umfaßt.

5. Expressionskassette nach einem vorangehenden Anspruch, worin das regulatorische Element Intron 3 des GADD45α-Gens umfaßt.

6. Expressionskassette nach Anspruch 5, worin das regulatorische Element ein mutmaßliches AP-1-Motiv umfaßt.

7. Expressionskassette nach einem vorangehenden Anspruch, worin die DNA-Sequenz ein Licht emittierendes Reporterprotein kodiert.

8. Expressionskassette nach einem vorangehenden Anspruch, worin die DNA-Sequenz grün fluoreszierendes Protein (GFP) und Licht emittierende Derivate davon kodiert.

9. Expressionskassette nach einem vorangehenden Anspruch, worin die DNA-Sequenz menschlich verstärktes GFP (hEGFP) kodiert, das aus dem Wild-Typ GFP besteht, enthaltend eine doppelte Aminosäuresubstitution von Phe-64 zu Leu und Ser-65 zu Thr.

10. Expressionskassette GD531, wie in Figur 9 dargestellt und wie in SEQ ID Nr. 2 bereitgestellt.

11. Expressionskassette GD532, wie in Figur 9 dargestellt und wie in SEQ ID Nr. 3 bereitgestellt.

12. Expressionskassette GD533, wie in Figur 9 dargestellt und wie in SEQ ID Nr. 4 bereitgestellt.

13. Rekombinanter Vektor, umfassend eine Expressionskassette nach einem der Ansprüche 1-12.

14. Rekombinanter Vektor nach Anspruch 13, worin der Vektor DNA des pCEP4-Plasmids umfaßt.

15. Rekombinanter Vektor PEP-GD531, wie in Figur 5 dargestellt.

16. Rekombinanter Vektor PEP-GD532, wie in Figur 6 dargestellt.

17. Rekombinanter Vektor PEP-GD533, wie in Figur 7 dargestellt.

18. Zelle, enthaltend einen rekombinanten Vektor nach einem der Ansprüche 14 - 17.

19. Zelle nach Anspruch 18, worin die Zelle eine menschliche Zelle ist.

20. Zelle nach Anspruch 19, worin die Zelle eine menschliche Zelle ist, die ein voll funktionales p53 aufweist.

21. Zelle nach Anspruch 20, worin die Zelle eine menschliche TK6-Zelllinie ist.

22. In vitro-Verfahren zum Detektieren der Anwesenheit eines Agens, das eine DNA-Schädigung verursacht oder verstärkt, umfassend die Schritte, daß eine Zelle nach einem der Ansprüche 18 - 21 einem Agens ausgesetzt wird und die Expression des Reporterproteins von der Zelle beobachtet wird.

23. Verfahren nach Anspruch 22, worin das Agens weiter gescreent und bewertet wird, ob es sicher ist, einen lebenden Organismus dem Agens auszusetzen.

24. Verfahren nach Anspruch 22 oder Anspruch 23, worin das Agens ein Medikamenten-Kandidat, ein Nahrungsmittelzusatz oder ein Kosmetikum ist.

25. Verfahren nach einem der Ansprüche 22 - 24, worin die Expression eines Licht emittierenden Reporterproteins von der Zelle beobachtet wird.

26. Verfahren nach Anspruch 25, worin das Licht emittierende Reporterprotein grün fluoreszierendes Protein ist.

27. Verfahren nach Anspruch 26, umfassend das Wachsen lassen von Zellen, die mit einem rekombinanten Vektor nach einem der Ansprüche 14 - 18 transfiziert sind, Inkubieren der Zellen mit dem Agens für eine vorbestimmte Zeit und Beobachten der Expression des grün fluoreszierenden Proteins direkt aus einer Probe der Zelle.

28. Verfahren nach einem der Ansprüche 22 - 27, worin die Zellen in einem niedrig fluoreszierenden Wachstumsmedium gewachsen sind.

29. Verfahren nach Anspruch 28, worin das niedrig fluoreszierende Wachstumsmedium ein RPMI-Medium ist, das kein Riboflavin enthält.

30. Verfahren nach Anspruch 28 oder 29, worin das niedrig fluoreszierende Wachstumsmedium ein RPMI-Medium ist, das kein Phenolrot enthält.

31. Verfahren nach einem der Ansprüche 25 - 30, worin die Fluoreszenz bestimmt wird durch Vergleichen der Fluoreszenz einer Zelle nach den Ansprüchen 18 - 21 mit der Fluoreszenz einer Kontrollzelle, umfassend einen Vektor, in dem die Nukleinsäure, die den Licht emittierenden Reporter kodiert, außerhalb des Rahmens plaziert ist.

## Revendications

1. Cassette d'expression comprenant une séquence d'ADN codant pour une protéine rapporteur, laquelle séquence d'ADN est liée de manière fonctionnelle à un promoteur du gène GADD45α humain et à un élément régulateur supplémentaire du gène GADD45α humain, la séquence d'ADN étant placée en 3' par rapport au promoteur du gène GADD45α, **caractérisée en ce que** l'élément régulateur comprend une région de l'intron 3 du gène GADD45α y compris un motif putatif de liaison de p53.

2. Cassette d'expression selon la revendication 1, dans laquelle l'élément régulateur comprend l'exon 1, l'exon 2, l'exon 3 et/ou l'exon 4 du gène GADD45α, ou une région de ceux-ci, ou toute combinaison de ceux-ci.

3. Cassette d'expression selon la revendication 2, dans laquelle l'élément régulateur comprend une région de l'exon 1 du gène GADD45α, une région de l'exon 3 du gène GADD45α, et une région de l'exon 4 du gène GADD45α.

4. Cassette d'expression selon l'une quelconque des revendications précédentes, dans laquelle l'élément régulateur comprend en outre l'intron 1 et/ou l'intron 2 du gène GADD45α, ou une région de ceux-ci.

5. Cassette d'expression selon l'une quelconque des revendications précédentes, dans laquelle l'élément régulateur comprend l'intron 3 du gène GADD45α.

6. Cassette d'expression selon la revendication 5, dans laquelle l'élément régulateur comprend un motif putatif AP-1.

7. Cassette d'expression selon l'une quelconque des revendications précédentes, dans laquelle la séquence d'ADN code pour une protéine rapporteur émettant de la lumière.

8. Cassette d'expression selon l'une quelconque des revendications précédentes, dans laquelle la séquence d'ADN code pour la protéine fluorescente verte (GFP), et des dérivés de celle-ci émettant de la lumière.

9. Cassette d'expression selon l'une quelconque des revendications précédentes, dans laquelle la séquence d'ADN code pour une GFP humaine amplifiée (hEGFP), qui consiste en la GFP de type sauvage contenant une double substitution d'acides aminés de Phe-64 en Leu et Ser-65 en Thr.

10. Cassette d'expression GD531 telle qu'illustrée sur la figure 9, et telle que représentée par SEQ ID NO : 2.

11. Cassette d'expression GD532 telle qu'illustrée sur la figure 9, et telle que représentée par SEQ ID NO : 3.

12. Cassette d'expression GD533 telle qu'illustrée sur la figure 9, et telle que représentée par SEQ ID NO : 4.

13. Vecteur recombinant comprenant une cassette d'expression selon l'une quelconque des revendications 1 à 12.

14. Vecteur recombinant selon la revendication 13, où le vecteur comprend de l'ADN provenant du plasmide pCEP4.

15. Vecteur recombinant PEP-GD531, tel qu'illustré sur la figure 5.

16. Vecteur recombinant PEP-GD532, tel qu'illustré sur la figure 6.

17. Vecteur recombinant PEP-GD533, tel qu'illustré sur la figure 7.

18. Cellule contenant un vecteur recombinant selon l'une quelconque des revendications 14 à 17.

19. Cellule selon la revendication 18, où la cellule est une cellule humaine.

20. Cellule selon la revendication 19, où la cellule est une cellule humaine renfermant une p53 totalement fonctionnelle.

21. Cellule selon la revendication 20, où la cellule est une lignée cellulaire humaine TK6.

22. Procédé *in vitro* de détection de la présence d'un agent qui provoque ou potentialise des dommages de l'ADN comprenant la soumission d'une cellule selon l'une quelconque des revendications 18 à 21 à un agent ; et le contrôle de l'expression de la protéine rapporteur à partir de la cellule.

23. Procédé selon la revendication 22, dans lequel l'agent est en outre criblé pour estimer s'il est sans risque d'exposer un organisme vivant à l'agent.

24. Procédé selon l'une ou l'autre de la revendication 22 ou de la revendication 23, dans lequel l'agent est un médicament candidat, un additif alimentaire ou un produit cosmétique.

25. Procédé selon l'une quelconque des revendications 22 à 24, dans lequel l'expression d'une protéine rapporteur émettant de la lumière à partir de la cellule est contrôlée.

26. Procédé selon la revendication 25, dans lequel la protéine rapporteur émettant de la lumière est la protéine fluorescente verte.

27. Procédé selon la revendication 26, comprenant la culture de cellules transfectées avec un vecteur recombinant selon l'une quelconque des revendications 14 à 18, l'incubation des cellules avec l'agent pendant un temps prédéterminé, et le contrôle de l'expression de la protéine fluorescente verte directement à partir d'un échantillon des cellules.

28. Procédé selon l'une quelconque des revendications 22 à 27, dans lequel les cellules sont cultivées dans un milieu de croissance à faible fluorescence.

29. Procédé selon la revendication 28, dans lequel le milieu de croissance à faible fluorescence est un milieu RPMI ne contenant pas de riboflavine.

30. Procédé selon la revendication 28 ou 29, dans lequel le milieu de croissance à faible fluorescence est un milieu RPMI ne contenant pas de rouge phénol.

31. Procédé selon l'une quelconque des revendications 25 à 30, dans lequel la fluorescence est déterminée par la comparaison de la fluorescence provenant d'une cellule selon les revendications 18 à 21 avec la fluorescence provenant d'une cellule contrôle comprenant un vecteur dans lequel l'acide nucléique codant pour le rapporteur émettant de la lumière est placé hors du cadre.
